# EUROPEAN PATENT APPLICATION

(11) **EP 4 692 122 A1**
(43) Date of publication of application: **11.02.2026**
(21) Application number: 24781085.6
(22) Date of filing: 18.03.2024
(51) Int. Cl.: C07K 16/28, A61K 47/68, C07K 14/725, C12N 5/0783, A61K 39/00, A61P 35/00, G01N 33/574

(54) **HUMANIZED ANTIBODIES TARGETING EPHA10**

(30) Priority: 24.03.2023 KR 20230038802
(71) Applicant: Korea University Research and Business Foundation, Seoul 02841 (KR); AJOU UNIVERSITY INDUSTRY-ACADEMIC COOPERATION FOUNDATION, Gyeonggi-do 16499 (KR)
(72) Inventor: JUNG, Sang-Taek, Seoul 06217 (KR); LEE, Ji-Sun, Seoul 03472 (KR); HAN, Ga-Ram, Changwon-si Gyeongsangnam-do 51736 (KR); CHA, Jong-Ho, Incheon 22000 (KR); SHIN, Ji-Soo, Bucheon-si Gyeonggi-do 14734 (KR); KIM, Min-Ji, Incheon 22188 (KR); YOO, Tae Hyeon, Yongin-si Gyeonggi-do 17003 (KR); LEE, Sangwoo, Jeju-si Jeju-do 63067 (KR); LEE, Suhyeon, Suwon-si Gyeonggi-do 16509 (KR)
(74) Representative: Isarpatent
(86) International application number: PCT/KR2024/003367
(87) International publication number: WO 2024/205094

(57) **Abstract**

The present invention relates to humanized antibodies targeting human EphA10 and use thereof. An antibody specific to EphA10 or an immunologically active fragment thereof according to the present disclosure specifically binds to EphA10, a highly promising target for overcoming resistance and non-responsiveness to existing immune checkpoint inhibitor therapies. When created with the antibody, an ADC was found to specifically kill cancer cells. Therefore, it can be used as a therapeutic antibody for cancer treatment leveraging cancer cell death mechanisms via immune cell activation or for treating EphA10-expressing cancers, or as a cell therapeutic for treating EphA10-expressing cancers, and can also be used for cancer diagnosis.

## Description

### [Technical Field]

The present disclosure relates to humanized antibodies targeting human EphA10 and uses thereof.

### [Background Art]

Cancer is a condition in which the proliferation and death of cells are not normally controlled by various changes in gene expression, and the abnormal growth of cells is caused, and infiltrates and destroys adjacent tissues, metastasizes to other regions, and eventually leads to death. The cause of cancer, that is, a mechanism through which normal cells are transformed into cancer cells, has not been precisely identified, but as far as it is concerned, it has been known that external factors such as environmental factors, chemicals, radiation, and viruses, internal factors such as genetic factors and immunological factors, and the like are complicatedly involved to cause the cancer. The cancer is largely classified into blood cancer showing abnormality in the number of blood cells and solid cancer in the form of a mass of cells having predetermined hardness and shape in the body. The cancer may occur in almost all parts of the body, such as blood and tissue, and examples thereof may include lung cancer, gastric cancer, breast cancer, oral cancer, liver cancer, uterine cancer, esophageal cancer, skin cancer, and the like. In the treatment of cancer, main methods include most surgery or radiation therapy and chemotherapy using chemotherapeutic agents that inhibit cell proliferation. Medicines for cancer treatment are largely divided into low molecular medicines and high molecular medicines, and the high molecular medicines with specificity are in the spotlight as therapeutic agents compared to the low molecular medicines with relatively large side effects without specificity.

Protein therapeutics are widely used in clinical trials by rapidly replacing non-specific low molecular compound therapeutics due to very high specificity for disease targets and low side effects and toxicity. Among protein therapeutics currently used in clinical trials, antibody therapeutics and Fc-fusion protein therapeutics fused with the Fc region of an antibody make up the largest part. Therapeutic antibodies are considered as one of the most effective cancer therapies by exhibiting very high target specificity compared to conventional low molecular drugs, and having not only low biotoxicity and few side effects, but also an excellent blood half-life of about 3 weeks. In fact, large pharmaceutical companies and research institutes around the world are accelerating the research and development of therapeutic antibodies that specifically bind to cancer cells, including cancer-causing factors, to effectively remove the cancer cells. Companies for developing therapeutic antibody drugs mainly consist of pharmaceutical companies, such as Roche, Amgen, Johnson & Johnson, Abbott, and BMS. Particularly, Roche includes representative products of Herceptin, Avastin, Rituxan, and the like for anticancer treatment, and not only produces large profits, achieving sales of approximately $19.5 billion in the global market in 2012 with these three therapeutic antibodies, but also leads the global antibody drug market. Johnson & Johnson, which developed Remicade, is also growing rapidly in the global antibody market due to increased sales, and pharmaceutical companies such as Abbott and BMS are also known to have many therapeutic antibodies in the final stages of development. As a result, in the global pharmaceutical market, where low molecular drugs had been dominated, the low molecular drugs have been rapidly replaced with biopharmaceuticals including therapeutic antibodies that are specific to disease targets and have low side effects.

Cancer cells express, on a cell surface, an immune checkpoint protein, which is used when normal cells suppress immune cell activation in order to evade a killing mechanism by immune cells, and recently, research on an immune checkpoint inhibitor has been actively conducted as a method for treating cancer (Immunity. 2018 Mar 20;48(3):434-452.). The immune checkpoint inhibitor is a drug that attacks cancer cells by blocking the activity of immune checkpoint proteins involved in T cell suppression to activate T cells, and representatively, antibodies for recognizing CTLA 4, PD-1, PD-L1 and the like are used. However, the response rate of cancer patients to cancer immunotherapy, including immune checkpoint inhibitors, immune cell therapy, therapeutic antibodies, and anticancer vaccines, still remains at the level of 15 to 45%, and most solid tumors have a problem of acquiring non-responsiveness and resistance to cancer immunotherapy. Accordingly, in previous studies utilizing mouse models, human cancer cell lines, and the like that were induced to acquire resistance to anti-PD-1 therapy by repeated application of anti-PD-1 antibody therapy, EphA10, a cell membrane protein, has been identified as a factor that causes non-responsiveness to therapy targeting immune checkpoint inhibitors. EphA10 is a type of Ephrin receptor, which is the largest family of receptor tyrosine kinases (RTKs). Unlike other Ephrin receptors, EphA10 is a very novel receptor whose function has been little studied. It has been reported that the expression level of EphA10 is greatly increased in all breast cancers, including Triple Negative Breast Cancer (TNBC), which has no appropriate therapy and a very poor prognosis, and significantly increased even in lung cancer, pancreatic cancer, prostate cancer, gastric cancer, thyroid cancer, and the like compared to normal tissues.

### [Disclosure]

### [Technical Problem]

An object of the present disclosure is to provide an antibody specific to EphA10 or an immunologically active fragment thereof.

In addition, another object of the present disclosure is to provide an antibody-drug conjugate specific to EphA10.

In addition, yet another object of the present disclosure is to provide a bispecific or multispecific antibody.

In addition, still another object of the present disclosure is to provide a chimeric antigen receptor (CAR).

In addition, still another object of the present disclosure is to provide a chimeric antigen receptor expressing cell.

In addition, still another object of the present disclosure is to provide a T-cell engager.

In addition, still another object of the present disclosure is to provide a pharmaceutical composition for preventing or treating cancer.

In addition, still another object of the present disclosure is to provide a composition for diagnosing cancer.

In addition, still another object of the present disclosure is to provide a use for preventing or treating cancer.

In addition, still another object of the present disclosure is to provide a method for treating cancer.

In addition, still another object of the present disclosure is to provide a use for diagnosing cancer.

### [Technical Solution]

In order to solve the object, an aspect of the present disclosure provides an antibody specific to EphA10 or an immunologically active fragment thereof.

In addition, another aspect of the present disclosure provides an antibody-drug conjugate specific to EphA10 including the antibody or the immunologically active fragment thereof.

In addition, yet another aspect of the present disclosure provides a bispecific or multispecific antibody including the antibody or the immunologically active fragment thereof.

In addition, still another aspect of the present disclosure provides a chimeric antigen receptor including the antibody or the immunologically active fragment thereof.

In addition, still another aspect of the present disclosure provides a chimeric antigen receptor expressing cell.

In addition, still another aspect of the present disclosure provides a T cell engager including the antibody or the immunologically active fragment thereof.

In addition, still another aspect of the present disclosure provides a pharmaceutical composition for preventing or treating cancer.

In addition, still another aspect of the present disclosure provides a composition for diagnosing cancer.

In addition, still another aspect of the present disclosure provides a use for the prevention or treatment of cancer of the antibody or the immunologically active fragment thereof, the antibody-drug conjugate, the bispecific or multispecific antibody, the chimeric antigen receptor expressing cell, or the T cell engager of the present disclosure.

In addition, still another aspect of the present disclosure provides a method for treating cancer including administering, to a subject suffering from cancer, the antibody or the immunologically active fragment thereof, the antibody-drug conjugate, the bispecific or multispecific antibody, the chimeric antigen receptor expressing cell, or the T cell engager of the present disclosure in a pharmaceutically effective amount.

In addition, still another aspect of the present disclosure provides a use for the diagnosis of cancer of the antibody or the immunologically active fragment thereof, the antibody-drug conjugate, the bispecific or multispecific antibody, the chimeric antigen receptor expressing cell, or the T cell engager of the present disclosure.

### [Advantageous Effects]

According to the present disclosure, the antibody specific to EphA10 or the immunologically active fragment thereof specifically binds to EphA10, a highly promising target for overcoming resistance and non-responsiveness to existing immune checkpoint inhibitor targeting therapies, and when created with the antibody or the immunologically active fragment thereof, an ADC was found to specifically kill cancer cells. Therefore, the ADC can be used as antibody therapies for cancer treatment using cancer cell death mechanisms by immune cell activation or for treating EphA10-expressing cancers, or as cell therapies for treating EphA10-expressing cancers, and can also be used for cancer diagnosis.

### [Description of Drawings]

FIG. 1 is a diagram illustrating analyzing the sizes and purity of 10 types of EphA10 antigen proteins expressed and purified in animal cells using SDS-PAGE gel.
FIG. 2 illustrates results of analyzing an amino acid sequence of an EphA10 target mouse antibody and results of analyzing the binding activity to EphA10 of a chimeric #2 antibody produced based on the amino acid sequencing result.
FIG. 3 is a diagram illustrating an amino acid sequence and antibody information of an EphA10 targeting humanized antibody.
FIG. 4 is a diagram illustrating results of analyzing the expression levels of EphA10 target humanized antibodies after expressed and purified in animal cells and confirming the size and purity of the purified humanized antibodies using an SDS-PAGE gel.
FIG. 5 illustrates results of ELISA analysis of the binding activity to EphA10 of five types of EphA10-targeting humanized antibodies.
FIG. 6 is a diagram confirming the binding activity to EphA10 of five types of EphA10-targeting humanized antibodies expressed in human and mouse cells.
FIG. 7 is a diagram illustrating results of quantitative binding constant analysis of EphA10 targeting humanized antibodies to EphA10.
FIG. 8 illustrates results of analyzing antibody-drug binding of ADCs produced with EphA10-specific antibodies 1-1 and 1-2 selected due to high binding activity.
FIG. 9 is a diagram confirming the cancer cell killing efficacy of ADCs produced with EphA10-specific antibodies 1-1 and 1-2.
FIG. 10 is a diagram confirming free cysteine residues present in the light chains by analyzing sequences of EphA10-specific humanized antibodies.
FIG. 11 is a diagram illustrating results of ELISA analysis of the binding activity to EphA10 of EphA10-specific humanized antibody 1-1 and EphA10-targeting ADC antibody 1-1 (ADC) produced using the EphA10-specific humanized antibody 1-1:
   1-1: EphA10-specific humanized antibody; and
   1-1 (ADC): EphA10-targeting ADC antibody
FIG. 12 is a diagram illustrating the sizes and purity of EphA10-specific humanized antibodies with improved ADC efficacy, in which free cysteine residues present in the light chains of EphA10-specific antibodies 1-1 and 1-2 are substituted with alanine (A) or serine (S), respectively, after expressed and purified in animal cells, by SDS-PAGE gel.
FIG. 13 illustrates results of analyzing the binding activity to EphA10 of EphA10-specific humanized antibodies with improved ADC efficacy.
FIG. 14 is a diagram illustrating construction of a yeast library and screening strategy for increasing the binding activity of EphA10 targeting humanized antibodies.
FIG. 15 is a diagram illustrating an iterative screening process using a flow cytometer to increase the binding activity of EphA10 targeting humanized antibodies.
FIG. 16 illustrates amino acid sequence analysis results of 10 types of antibodies A.3, A.7, A.8, B.1, B.3, B.7, 2.1, 2.3, 1-N and 2-W discovered through exploration using a flow cytometer and 6 types of humanized antibodies 1-R, 1-W, 1-Y, 2-N, 2-R and 2-Y with additional free cysteines substituted.
FIG. 17 is a diagram illustrating the sizes and purity of 16 humanized antibodies with increased binding activity to EphA10 after expressed and purified in animal cells, by SDS-PAGE gel.
FIG. 18 illustrates results of analyzing the binding activity to EphA10 of 16 types of humanized antibodies with increased binding activity to EphA10.
FIG. 19 illustrates results of analyzing the antibody-drug binding of ADCs produced with two types A7 and 2W among 16 types of humanized antibodies with increased binding activity to EphA10.
FIG. 20 is a diagram illustrating results of ELISA analysis of the binding activity to EphA10 of EphA10-specific antibody 1-1, ADC 1-1_ADC produced using the EphA10-specific antibody 1-1, and humanized antibodies A7 and 2W with increased binding activity to EphA10, and EphA10-targeting ADC antibodies A7_ADC and 2W_ADC produced using the humanized antibodies A7 and 2W.
FIG. 21 is a diagram confirming the cancer cell killing efficacy of A7_ADC and 2W_ADC.
FIG. 22 is a diagram illustrating results of quantitative binding constant analysis of EphA10 of humanized antibodies A7 and 2W with increased binding activity to EphA10.
FIG. 23 is a diagram illustrating the sizes and purity of fusion proteins containing four types of mouse EphA10 antigens after expressed and purified in animal cells by SDS-PAGE gel, and ELISA analysis of the activity thereof.
FIG. 24 is a diagram illustrating results of ELISA analysis of the binding activity to mouse EphA10 of humanized antibodies A7 and 2W with increased binding activity to EphA10.

### [Best Mode]

Hereinafter, embodiments of the present disclosure will be described in detail with reference to the accompanying drawings. However, the following embodiments are presented as examples for the present disclosure, and when it is determined that the detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used in the present disclosure are terminologies used to properly express preferred examples of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be understood to imply the inclusion of stated elements, not the exclusion of any other elements.

All technical terms used in the present disclosure, unless otherwise defined, are used as the meaning as commonly understood by those skilled in the related art of the present disclosure. In addition, although preferred methods and samples are described in the present disclosure, similar or equivalent methods and samples thereto are also included in the scope of the present disclosure. The contents of all publications described in the present disclosure as references are incorporated in the present disclosure.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned in the present disclosure as abbreviations are also described as follows according to the IUPAC-IUB nomenclature.

Alanine: A; Arginine: R; Asparagine: N; Aspartic acid: D; Cysteine: C; Glutamic acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y; and Valine: V.

In one aspect, the present disclosure relates to an antibody specific to Eph receptor A10 (EphA10) or an immunologically active fragment thereof.

In one embodiment, EphA10 may be human EphA10 or mouse EphA10.

In one embodiment, the antibody or the immunologically active fragment thereof of the present disclosure may specifically bind to an extra cellular domain (ECD) of EphA10 or a FNIII domain of the ECD.

In an embodiment, the antibody or the immunologically active fragment thereof of the present disclosure may include a VH domain including a complementarity determining regions heavy chain (CDRH)1 including any one selected from the group consisting of amino acid sequences represented by SEQ ID NOS: 1 to 3, a CDRH2 including any one selected from the group consisting of amino acid sequences represented by SEQ ID NOS: 4 to 6, and a CDRH3 including an amino acid sequence represented by SEQ ID NO: 7 or 8.

In one embodiment, the antibody or the immunologically active fragment thereof of the present disclosure may include a VL domain including a CDRL1 including any one selected from the group consisting of amino acid sequences represented by SEQ ID NOS: 9 to 14, a CDRL2 including an amino acid sequence represented by SEQ ID NO: 15 or 16, and a CDRL3 including any one selected from the group consisting of amino acid sequences represented by SEQ ID NOS: 17 to 19.

In an embodiment, the antibody or the immunologically active fragment thereof of the present disclosure may include a VH domain including any one selected from the group consisting of amino acid sequences represented by SEQ ID NOS: 20 to 25.

In an embodiment, the antibody or the immunologically active fragment thereof of the present disclosure may include a VL domain including any one selected from the group consisting of amino acid sequences represented by SEQ ID NOS: 26 to 45.

In one embodiment, an antibody 1-1 of the present disclosure may include a VH domain including an amino acid sequence represented by SEQ ID NO: 20 and a VL domain including an amino acid sequence represented by SEQ ID NO: 26.

In one embodiment, an antibody 1-2 of the present disclosure may include a VH domain including an amino acid sequence represented by SEQ ID NO: 20 and a VL domain including an amino acid sequence represented by SEQ ID NO: 27.

In one embodiment, an antibody A.3 of the present disclosure may include a VH domain including an amino acid sequence represented by SEQ ID NO: 21 and a VL domain including an amino acid sequence represented by SEQ ID NO: 28.

In one embodiment, an antibody A.7 of the present disclosure may include a VH domain including an amino acid sequence represented by SEQ ID NO: 20 and a VL domain including an amino acid sequence represented by SEQ ID NO: 29.

In one embodiment, an antibody A.8 of the present disclosure may include a VH domain including an amino acid sequence represented by SEQ ID NO: 20 and a VL domain including an amino acid sequence represented by SEQ ID NO: 30.

In one embodiment, an antibody B.1 of the present disclosure may include a VH domain including an amino acid sequence represented by SEQ ID NO: 22 and a VL domain including an amino acid sequence represented by SEQ ID NO: 31.

In one embodiment, an antibody B.3 of the present disclosure may include a VH domain including an amino acid sequence represented by SEQ ID NO: 23 and a VL domain including an amino acid sequence represented by SEQ ID NO: 32.

In one embodiment, an antibody B.7 of the present disclosure may include a VH domain including an amino acid sequence represented by SEQ ID NO: 24 and a VL domain including an amino acid sequence represented by SEQ ID NO: 33.

In one embodiment, an antibody 2.1 of the present disclosure may include a VH domain including an amino acid sequence represented by SEQ ID NO: 25 and a VL domain including an amino acid sequence represented by SEQ ID NO: 34.

In one embodiment, an antibody 2.3 of the present disclosure may include a VH domain including an amino acid sequence represented by SEQ ID NO: 20 and a VL domain including an amino acid sequence represented by SEQ ID NO: 35.

In one embodiment, an antibody 1-A of the present disclosure may include a VH domain including an amino acid sequence represented by SEQ ID NO: 20 and a VL domain including an amino acid sequence represented by SEQ ID NO: 36.

In one embodiment, an antibody 1-N of the present disclosure may include a VH domain including an amino acid sequence represented by SEQ ID NO: 20 and a VL domain including an amino acid sequence represented by SEQ ID NO: 37.

In one embodiment, an antibody 1-R of the present disclosure may include a VH domain including an amino acid sequence represented by SEQ ID NO: 20 and a VL domain including an amino acid sequence represented by SEQ ID NO: 38.

In one embodiment, an antibody 1-W of the present disclosure may include a VH domain including an amino acid sequence represented by SEQ ID NO: 20 and a VL domain including an amino acid sequence represented by SEQ ID NO: 39.

In one embodiment, an antibody 1-Y of the present disclosure may include a VH domain including an amino acid sequence represented by SEQ ID NO: 20 and a VL domain including an amino acid sequence represented by SEQ ID NO: 40.

In one embodiment, an antibody 2-A of the present disclosure may include a VH domain including an amino acid sequence represented by SEQ ID NO: 20 and a VL domain including an amino acid sequence represented by SEQ ID NO: 41.

In one embodiment, an antibody 2-W of the present disclosure may include a VH domain including an amino acid sequence represented by SEQ ID NO: 20 and a VL domain including an amino acid sequence represented by SEQ ID NO: 42.

In one embodiment, an antibody 2-N of the present disclosure may include a VH domain including an amino acid sequence represented by SEQ ID NO: 20 and a VL domain including an amino acid sequence represented by SEQ ID NO: 43.

In one embodiment, an antibody 2-R of the present disclosure may include a VH domain including an amino acid sequence represented by SEQ ID NO: 20 and a VL domain including an amino acid sequence represented by SEQ ID NO: 44.

In one embodiment, an antibody 2-Y of the present disclosure may include a VH domain including an amino acid sequence represented by SEQ ID NO: 20 and a VL domain including an amino acid sequence represented by SEQ ID NO: 45.

In one embodiment, the antibody or the immunologically active fragment thereof of the present disclosure may include a heavy or light chain variable region, a heavy or light chain constant region, a framework region, or any moiety thereof derived from a human.

In an embodiment, the immunologically active fragment may be any one selected from the group consisting of Fab, Fd, Fab', dAb, F(ab'), F(ab')₂, single chain fragment variable (scFv), Fv, a single chain antibody, a Fv dimmer, a complementarity determining region fragment, a humanized antibody, a chimeric antibody, and a diabody, and more preferably a humanized antibody, Fab, or scFv.

In one embodiment, the antibody of the present disclosure may be a monoclonal antibody.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

The antibody or the immunologically active fragment thereof of the present disclosure may be selected from the group consisting of animal-derived antibodies, chimeric antibodies, humanized antibodies, human antibodies, and immunologically active fragments thereof. The antibody may be produced recombinantly or synthetically.

Animal-derived antibodies produced by immunizing an animal to be immunized with a desired antigen may generally cause immune rejection when administered to humans for treatment, and chimeric antibodies have been developed to suppress such immune rejection. The chimeric antibody is obtained by substituting a constant region of an animal-derived antibody, which causes an anti-isotype response, with a constant region of a human antibody using a genetic engineering method. Compared to animal-derived antibodies, the chimeric antibody is improved significantly in the anti-isotype response, but includes side effects on potential anti-idiotypic responses because animal-derived amino acids are still present in a variable region. The humanized antibody is developed to improve these side effects. The humanized antibody is produced by grafting, to a human antibody framework, complementarity determining regions (CDRs), which play an important role in binding to the antigen among the variable regions of the chimeric antibody.

In the CDR grafting technology for producing the humanized antibody, it is most important to select an optimized human antibody that may best accept the CDR region of the animal-derived antibody, and to this end, applications of an antibody database, analysis of a crystal structure, molecular modeling technology, and the like are used. However, even if the CDR region of the animal-derived antibody is grafted into an optimized human antibody framework, in some cases, there are amino acids that affect binding to the antigen while being located in the framework of the animal-derived antibody. As a result, since there are many cases in which the antigen-binding activity is not conserved, it may be required to apply additional antibody engineering techniques to restore the antigen-binding activity.

The antibody or the immunologically active fragment thereof may be isolated from a living body (not present in a living body) or may non-naturally occur, and for example, may be synthetically or recombinantly produced.

In the present disclosure, the "antibody" refers to a substance produced by stimulation of an antigen in the immune system, and the type thereof is not particularly limited, and may be obtained naturally or non-naturally (e.g., synthetically or recombinantly). The antibody is advantageous for mass expression and production because of being very stable in vivo as well as ex vivo and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and has subclasses of gamma 1 (γ1), gamma 2 (γ2), gamma 3 (γ3), gamma 4 (γ4), alpha 1 (α1) and alpha 2 (α2). The light chain constant region has kappa (κ) and lambda (λ) types.

In the present disclosure, the term "heavy chain" is interpreted as a meaning including all of a full-length heavy chain including a variable region domain V_{H} including an amino acid sequence having a sufficient variable region sequence to impart specificity to an antigen, three constant region domains C_{H1}, C_{H2} and C_{H3} and hinges, and fragments thereof. In addition, the term "light chain" is interpreted as a meaning including all of a full-length light chain including a variable region domain V_{L} including an amino acid sequence having a sufficient variable region sequence to impart specificity to an antigen and a constant region domain C_{L}, and fragments thereof.

In the present disclosure, the term "variable region or variable domain" refers to a part of an antibody molecule that exhibits many variations on the sequence while performing a function that specifically binds to an antigen, and in the variable region, there are complementarity determining regions CDR1, CDR2 and CDR3. A framework region (FR) portion exists between the CDRs to support a CDR ring. The "complementarity determining region" is a ring-shaped domain involved in antigen recognition, and the specificity of the antibody to the antigen is determined as the sequence of this domain changes.

In the present disclosure, the term "single chain fragment variable (scFv)" refers to a single-chain antibody formed by expressing only a variable region of the antibody through genetic recombination, and is a single-chain form in which the VH and VL regions of the antibody are linked to each other by a short peptide chain. The term "scFv" is intended to include a scFv fragment including an antigen-binding fragment, unless otherwise specified or otherwise understood from the context. This is obvious to those skilled in the art.

In the present disclosure, the term "complementarity determining region (CDR)" refers to an amino acid sequence of a hypervariable region of the heavy and light chains of immunoglobulin. The heavy and light chains may include three CDRs (CDRH1, CDRH2, CDRH3 and CDRL1, CDRL2, CDRL3), respectively. The CDRs may provide key contact residues for an antibody binding to an antigen or epitope.

In the present disclosure, the term "specifically binding" or "specifically recognizing" has the same meaning as commonly known to those skilled in the art, and means that an antigen and an antibody specifically interact with each other to cause an immunological response.

In the present disclosure, the term "antigen-binding fragment" refers to a fragment of the entire structure of immunoglobulin, and refers to a portion of a polypeptide including a portion capable of binding to the antigen. For example, the antigen-binding fragment may be scFv, (scFv)2, scFv-Fc, Fab, Fab' or F(ab')2, but is not limited thereto. The Fab of the antigen binding fragments has a structure having variable regions of the light and heavy chains, a constant region of the light chain, and a first constant region C_{H1} of the heavy chain, and has one antigen-binding site. The Fab' is different from Fab by having a hinge region containing one or more cysteine residues in a C-terminal of the heavy chain C_{H1} domain. The F(ab')2 antibody is produced when the cysteine residues in the hinge region of Fab' form disulfide bonds. Fv is a minimal antibody fragment having only a heavy chain variable region and a light chain variable domain, and a recombination technique for generating the Fv fragment is widely known in the art. The two-chain Fv has a heavy chain variable domain and a light chain variable domain linked by noncovalent bonds, and the single-chain Fv may generally form a dimer-like structure, such as the two-chain Fv because the variable region of the heavy chain and the variable region of the single chain are covalently linked via a peptide linker or directly linked at a C-terminal. The linker may be a peptide linker consisting of 1 to 100 or 2 to 50 any amino acids, and appropriate sequences are known in the art. The antigen binding fragments may be obtained using protease (for example, the whole antibody is restriction-cleaved with papain to obtain Fab, and cleaved with pepsin to obtain a F(ab')2 fragment), or may be produced through genetic recombination technology.

In the present disclosure, the term "hinge region" refers to a region included in the heavy chain of the antibody, and means a region which is present between the C_{H1} and C_{H2} regions and functions to provide flexibility of the antigen binding domain in the antibody. For example, the hinge may be derived from a human antibody, and specifically, derived from IgA, IgE, or IgG, such as IgG1, IgG2, IgG3, or IgG4.

In the present disclosure, the term "amino acid modification/mutation" refers to substitution, insertion and/or deletion, preferably substitution of amino acids in a polypeptide sequence. As used in the present disclosure, the term "amino acid substitution" or "substitution" means that an amino acid at a specific position in the polypeptide sequence of the antibody is replaced with another amino acid.

The antibody or the immunologically active fragment thereof of the present disclosure may be prepared by any method known in the art. After encoding a polypeptide sequence of the antibody or the immunologically active fragment thereof of the present disclosure, if desired, the polypeptide sequence is cloned into a host cell and used to form a nucleic acid to be expressed and assayed. Various methods therefor are described in the literature (Molecular Cloning-A Laboratory Manual, 3rd Ed., Maniatis, Cold Spring Harbor Laboratory Press, New York, 2001; Current Protocols in Molecular Biology, John Wiley & Sons).

A nucleic acid encoding the antibody or the immunologically active fragment thereof of the present disclosure may be inserted into an expression vector for protein expression. The expression vector generally includes a protein operably linked, i.e., functionally related to a control or regulatory sequence, a selectable marker, any fusion partner, and/or an additional element. Under appropriate conditions, the antibody or the immunologically active fragment thereof of the present disclosure may be produced by a method of inducing protein expression by culturing a host cell transformed with a nucleic acid, preferably an expression vector containing a nucleic acid encoding the antibody or the immunologically active fragment thereof according to the present disclosure. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, the antibody or the immunologically active fragment thereof according to the present disclosure is produced using E. coli as a host cell, which has low production cost and high industrial utility value.

Therefore, the scope of the present disclosure includes a method for producing an antibody specific to EphA10 or an immunologically active fragment thereof, including culturing a host cell into which a nucleic acid encoding the antibody or the immunologically active fragment thereof of the present disclosure has been introduced under conditions suitable for protein expression; and purifying or isolating the antibody or the immunologically active fragment thereof expressed from the host cell.

The antibody may be isolated or purified by various methods known in the art. A standard purification method includes chromatography, electrophoresis, immunology, precipitation, dialysis, filtration, concentration, and chromatofocusing techniques. As known in the art, various natural proteins such as bacterial proteins A, G, and L bind to the antibody and these proteins may be used for purification. Often, purification by specific fusion partners may be enabled.

In one aspect, the present disclosure relates to an antibody-drug conjugate (ADC) including an antibody specific to EphA10 or an immunologically active fragment thereof of the present disclosure, and a drug.

In one embodiment, the drug may be immunogenic apoptosis-inducing agents, pro-apoptotic peptides, microtubulin structure formation inhibitors, mitosis inhibitors, topoisomerase inhibitors, DNA intercalators, toxins, radionuclides or anticancer agents.

In one embodiment, the pro-apoptotic peptide may be selected from the group consisting of KLA, alpha-defensin-1, BMAP-28, Brevenin-2R, Buforin IIb, cecropin A-Magainin 2 (CA-MA-2), Cecropin A, Cecropin B, chrysophsin-1, D-K6L9, Gomesin, Lactoferricin B, LLL27, LTX-315, Magainin 2, Magainin II-bombesin conjugate (MG2B), Pardaxin, and combinations thereof.

In one embodiment, the immunogenic apoptosis-inducing agent may be selected from the group consisting of anthracycline-based anticancer agents, taxane-based anticancer agents, an anti-EGFR antibody, a BK channel agonist, Bortezomib, cardiac glycoside, cyclophosmide-based anticancer agents, a GADD34/PP1 inhibitor, LV-tSMAC, Measles virus, bleomycin, mitoxantrone, oxaliplatin, and combinations thereof.

In one embodiment, the anticancer agent may be selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof.

In one embodiment, the anticancer agent may be a cancer immunotherapy, and the cancer immunotherapy may be immune checkpoint inhibitors, an immunosuppressant control drug, a cancer vaccine, an immunoadjuvant, an immune cell for cancer treatment, an immune cell activation cofactor, an antibody for cancer treatment, or cytokines required to maintain the activity of an immune cell for cancer treatment.

In one embodiment, the immunosuppressant control drug may be a drug that reduces the level of regulatory T cells (Treg), and may reduce regulatory T cells to activate the immune response of T effector cells.

In one embodiment, the immune checkpoint inhibitor may be an inhibitor of CTLA-4, PD-1, PD-L1, PD-L2, LAG-3, BTLA, B7H3, B7H4, TIM3, KIR, TIGIT, CD47, VISTA or A2aR, and may be an anti-PD-1 antibody, an anti-PD-L1 antibody, an anti-CTLA-4 antibody, or a variant thereof.

In one embodiment, the drug may be selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof.

In one embodiment, in the antibody-drug conjugate, a drug may be linked/bound with the antibody or the immunologically active fragment thereof of the present disclosure via a linker, and the linker may be used with any linker having a functional group that may bind via an amine group, carboxyl group, or sulfhydryl group of a protein such as a peptide, a ligand, an antibody, or an antibody fragment, or a phosphate group or hydroxyl group of a nucleic acid such as an aptamer. The functional group of the linker may be isothiocyanate, isocyanates, acyl azide, NHS ester, sulfonyl chloride, aldehyde, glyoxal, epoxide, oxirane, carbonate, arylhalide, imidoester, carbodiimide, anhydride, fluorophenyl ester, hydroxymethyl phosphine, maleimide, haloacetyl, pyridyldisulfide, thiosulfonate, vinylsulfone, or the like. The linker may be a linker that is cleavable by protease, cleavable under acid or basic conditions, cleavable by high temperature or light irradiation, or cleavable under reducing or oxidizing conditions, or a linker that is not cleavable under these conditions. Examples of the cleavable linker may include hydrazone linkers that are cleaved under acidic conditions, peptide linkers that are cleaved by protease, linkers having a disulfide functional group cleaved under reducing conditions, and the like. Examples of the non-cleavable linker may include a maleimidomethyl cyclohexane-1-carboxylate (MCC) linker, a maleimidocaproyl (MC) linker, or derivatives thereof, such as succinimidyl-4-(N-maleimidomethyl)cyclohexane-1-carboxylate (sMCC) linker or sulfosuccinimidyl-4-(N-maleimidomethyl)cyclohexane-1-carboxylate (sulfo-sMCC).

In addition, the linker may be a self-immolative linker or a traceless linker after cleavage. The self-immolative linker may include, for example, linkers disclosed in U.S. Pat. No. 9,089,614 entitled "Hydrophilic self-immolative linkers and conjugates thereof", and linkers disclosed in International Publication No. WO2015038426 entitled "SELF-IMMOLATIVE LINKERS CONTAINING MANDELIC ACID DERIVATIVES, DRUG-LIGAND CONJUGATES FOR TARGETED THERAPIES AND USES THEREOF", and the traceless linker after cleavage may include a phenylhydrazide linker, an aryl-triazene linker, linkers disclosed in the literature [Blaney, et al., "Traceless solid-phase organic synthesis," Chem Rev. 102: 2607-2024 (2002)], and the like.

The antibody or the immunologically active fragment thereof of the present disclosure and the drug of the antibody-drug conjugate of the present disclosure may also be bound via a biocompatible polymer or carrier. The biocompatible polymer means a polymer that has tissue compatibility and blood compatibility, which does not cause tissue necrosis or blood coagulation when in contact with living tissue or blood. A synthetic polymer as the biocompatible polymer includes polyester, polyhydroxyalkanoate (PHA), poly(α-hydroxyacid), poly(β-hydroxyacid), poly(3-hydroxybutyrate-co-valerate (PHBV), poly(3-hydroxyproprionate (PHP), poly(3-hydroxyhexanoate (PHH), poly(4-hydroxyacid), poly(4-hydroxybutyrate), poly(4-hydroxyvalerate), poly(4-hydroxyhexanoate), poly(esteramide), polycaprolactone, polylactide, polyglycolide, poly(lactide-co-glycolide) (PLGA), polydioxanone, polyorthoester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHA-PEG, ethylene vinyl alcohol copolymer (EVOH), polyurethane, silicone, polyester, polyolefin, polyisobutylene and ethylene-alphaolefin copolymer, styrene-isobutylene-styrene triblock copolymer, acrylic polymer and copolymer, vinyl halide polymer and copolymer, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatics, polystyrene, polyvinyl ester, polyvinyl acetate, ethylene-methyl methacrylate copolymer, acrylonitrile-styrene copolymer, ABS resin and ethylene-vinyl acetate copolymer, polyamide, alkyd resin, polyoxymethylene, polyimide, polyether, polyacrylate, polymethacrylate, polyacrylic acid-co-maleic acid or polyaminoamine, and the natural polymer is chitosan, dextran, cellulose, heparin, hyaluronic acid, alginate, inulin, starch or glycogen.

In one embodiment, the antibody-drug conjugate may further include an ADC linker, and the ADC linker may be 6-maleimidocaproyl (MC), maleimidopropanoyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl) cyclohexane-1 carboxylate (SMCC), valine-citrulline-p-aminobenzyloxycarbonyl (val-cit-PAB), or N-succinimidyl (4-iodo-acetyl) aminobenzoate (SIAB).

In one embodiment, the antibody-drug conjugate may form a complex of the drug with the antibody specific to EphA10 or the immunologically active fragment thereof of the present disclosure via the ADC linker.

In one embodiment, the antibody specific to EphA10 or the immunologically active fragment thereof of the present disclosure may be further conjugated with RNA, DNA, an antibody, an effector, a drug, a prodrug, a toxin, a peptide or a delivery molecule (see Shoari et al., Pharmaceutics 13:1391, pp. 1-32 (2021)).

In one aspect, the present disclosure relates to a bispecific or multispecific antibody including an antibody specific to EphA10 or an immunologically active fragment thereof of the present disclosure, and a moiety binding to a target antigen other than EphA10.

In one embodiment, the moiety binding to the target antigen may include an antibody or an immunologically active fragment thereof.

In one embodiment, the target antigen may be at least one selected from the group consisting of 17-1A antigen, GD3 ganglioside R24, EGFRvIII, PSMA, PSCA, HLA-DR, EpCAM, MUC1 core protein, aberrant glycosylation MUC1, fibronectin heteroform containing ED-B domain, HER2/neu, carcinoembryonic antigen (CEA), gastrin-releasing peptide (GRP) receptor antigen, mucin antigen, epidermal growth factor receptor (EGF-R), HER3, HER4, MAGE antigen, SART antigen, MUC1 antigen, c-erb-2 antigen, TAG 72, carbonic anhydrase IX, alpha-fetoprotein, A3, an antigen specific to an A33 antibody, Ba 733, BrE3-antigen, CA125, CDl, CD1a, CD3, CD5, CDl5, CD16, CD19, CD20, CD21, CD22, CD23, CD25, CD30, CD33, CD38, CD40, CD45, CD52, CD74, CD79a, CD80, CD138, colon-specific antigen-p (CSAp), CSAp, EGP-1, EGP-2, Ep-CAM, FIt-1, Flt-3, folate receptor, HLA-DR, human chorionic gonadotropin (HCG) and its subunits, hypoxia inducible factor (HIF-I), la, IL-2, IL-6, IL-8, insulin growth factor-1 (IGF-1), KC4-antigen, KS-1-antigen, KSl-4, Le-Y, macrophage inhibitory factor (MIF), MAGE, MUC1, MUC2, MUC3, MUC4, NCA66, NCA95, NCA90, antigen specific to PAM-4 antibody, placental growth factor, p53, prostatic acid phosphatase, PSA, RS5, SlOO, TAC, tenascin, TRAIL receptors, Tn antigen, Thomson-Friedenreich antigens, tumor necrosis antigens, VEGF, ED-B fibronectin, angiogenesis marker, oncogene marker or oncogene product.

In an embodiment, the apoptosis-related gene may be ABL1, AKT1, AKT2, BARD1, BAX, BCL11B, BCL2, BCL2A1, BCL2L1, BCL2L12, BCL3, BCL6, BIRC2, BIRC3, BIRC5, BRAF, CARD11, CAV1, CBL, CDC25A, CDKN1A, CFLAR, CNR2, CTNNB1, CUL4A, DAXX, DDIT3, E2F1, E2F3, E2F5, ESPL1, FOXO1, HDAC1, HSPA5, IGF1R, IGF2, JUN, JUNB, JUND, MALT1, MAP3K7, MCL1, MDM2, MDM4, MYB, MYC, NFKB2, NPM1, NTRK1, PAK1, PAX3, PML, PRKCA, PRKCE, PTK2B, RAF1, RHOA, TGFB1, TNFRSF1B, TP73, TRAF6, YWHAG, YWHAQ or YWHAZ. The transcription factor gene may be AR, ARID3A, ASCL1, ATF1, ATF3, BCL11A, BCL11B, BCL3, BCL6, CDC5L, CDX2, CREB1, CUX1, DDIT3, DLX5, E2F1, E2F3, E2F5, ELF4, ELK1, ELK3, EN2, ERG, ETS1, ETS2, ETV1, ETV3, ETV4, ETV6, FEV, FEZF1, FLI1, FOS, FOSL1, FOXA1, FOXG1, FOXM1, FOXO1, FOXP1, FOXQ1, GATA1, GATA6, GFI1, GFI1B, GLI1, GLI2, GLI3, HES6, HHEX, HLF, HMGA1, HMGA2, HOXA1, HOXA9, HOXD13, HOXD9, ID1, ID2, IKZF1, IRF2, IRF4, JUN, JUNB, JUND, KAT6A, KDM2A, KDM5B, KLF2, KLF4, KLF5, KLF6, KLF8, KMT2A, LEF1, LHX1, LMX1B, MAF, MAFA, MAFB, MBD1, MECOM, MEF2C, MEIS1, MITF, MYB, MYC, MYCL, MYCN, NANOG, NCOA3, NFIB, NFKB2, NKX2-1, OTX2, PATZ1, PAX2, PAX3, PAX4, PAX8, PBX1, PBX2, PITX2, PLAG1, PLAGL2, PPARG, PPP1R13L, PRDM10, PRDM13, PRDM14, PRDM15, PRDM16, PRDM6, PRDM8, PRDM9, RARA, REL, RERE, RUNX1, RUNX3, SALL4, SATB1, SFPQ, SIX1, SNAI1, SOX2, SOX4, SPI1, SREBF1, STAT3, TAF1, TAL1, TAL2, TBX2, TBX3, TCF3, TFCP2, TFE3, THRA, TLX1, TP63, TP73, TWIST1, WT1, YBX1, YY1, ZBTB16, ZBTB7A, ZIC2, ZNF217 or ZNF268. The metastasis-related gene may be AKT1, AKT2, AR, CBL, CDH1, CRK, CSF1, CTNNB1, CTTN, CXCR4, EGFR, FGFR1, FLT3, FYN, GLI1, ILK, ITGA3, JAK2, MET, PDGFRB, PLXNB1, PRKCI, PTCH1, PTPN11, RAC1, RHOA, RHOC, ROCK1, SMO, SNAI1, SRC, TCF3 or WT1. The angiogenesis-related gene may be BRAF, CAV1, CTGF, EGFR, ERBB2, ETS1, FGF4, FGF6, FGFR1, FGFR3, FGFR4, ID1, NRAS, PDGFB, PDGFRA, PDGFRB or SPARC. The tyrosine-kinase gene may be ABL1, ABL2, ALK, AXL, BLK, EGFR, EPHA2, ERBB2, ERBB3, ERBB4, FES, FGFR1, FGFR2, FGFR3, FGFR4, FGR, FLT3, FYN, ITK, JAK1, JAK2, KIT, LCK, MERTK, MET, MST1R, NTRK1, NTRK3, PDGFRA, PDGFRB, PTK2B, PTK7, RET, ROS1, SRC, SYK, TEC or YES1.

In one embodiment, the oncogene may be at least one selected from the group consisting of SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703 and ZNHIT6.

In one embodiment, the target antigen may be a cell surface antigen or an autoantigen.

In one embodiment, the cell surface antigen may be at least one selected from the group consisting of CEA, ED-B fibronectin, CD20, CD22, CDl9, EGFR, IGFlR, VEFGRl/Flt-1, VEGFR2/KDR, VEGRF3/Flt-4, HER2/neu, CD30, CD33, CD3, CD16, CD64 , CD89, CD2, adenovirus fiber knob, PfMSP-1, HN/NDV, EpCAM/17-lA, hTR, IL-2R/Tac, CA1l-9, MUCl, HLA class II, GD2, G250, TAG-72, PSMA, CEACAM6, HMWMAA, CD40, Ml3 enveloped protein and GPIIb/IIIa.

In one aspect, the present disclosure relates to an isolated nucleic acid molecule encoding an antibody or an immunologically active fragment thereof of the present disclosure, or a bispecific or multispecific antibody of the present disclosure, a vector including the same and a host cell transformed therewith.

The nucleic acid molecule of the present disclosure may be isolated or recombined, and includes not only DNA and RNA in a single-stranded and double-stranded form, but also corresponding complementary sequences. The isolated nucleic acid is a nucleic acid that is isolated from surrounding genetic sequences present in the genome of a subject from which the nucleic acid is isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, DNA of a presequence or secretory leader is operably linked to DNA of the polypeptide when being expressed as a preprotein in the form before the polypeptide is secreted, a promoter or an enhancer is operably linked to a coding sequence when affecting the transcription of the polypeptide sequence, or a ribosome binding domain is operably linked to a coding sequence when disposed to promote the translation. In general, "operably linked" means that DNA sequences to be linked are located contiguously, and that the secretory leader exists contiguously in the same leading frame. However, the enhancer needs not to be contiguously located. The linkage is achieved by ligation at a convenient restriction enzyme site. When there is no site, synthetic oligonucleotide adapters or linkers are used according to conventional methods.

In the isolated nucleic acid molecule encoding the antibody or the immunologically active fragment thereof of the present disclosure, or the bispecific or multispecific antibody of the present disclosure, due to the degeneracy of codons or in consideration of codons preferred in an organism to express the antibody, it will be well understood by those skilled in the art that various modifications may be made to a coding region within a range without changing the amino acid sequence of the antibody to be expressed from a coding region, various modifications or changes may be made within a range without affecting the expression of the gene even in parts other than the coding region, and such modified genes are also included within the scope of the present disclosure. That is, as long as the nucleic acid molecule of the present disclosure encodes a protein having equivalent activity thereto, one or more nucleotides may be mutated by substitution, deletion, insertion, or a combination thereof, which are also included in the scope of the present disclosure. The sequence of such a nucleic acid molecule may be single- or double-stranded, and may be a DNA molecule or an RNA (mRNA) molecule.

The isolated nucleic acid molecule encoding the antibody or the immunologically active fragment thereof of the present disclosure or the bispecific or multispecific antibody of the present disclosure may be inserted into an expression vector for protein expression. The expression vector generally includes a protein operably linked, i.e., functionally related to a control or regulatory sequence, a selectable marker, any fusion partner, and/or an additional element. In appropriate conditions, the antibody or the immunologically active fragment thereof of the present disclosure or the bispecific or multispecific antibody of the present disclosure may be produced by a method for inducing the protein expression by culturing a host cell transformed with a nucleic acid, preferably an expression vector containing an isolated nucleic acid molecule encoding the antibody or the immunologically active fragment thereof of the present disclosure or the bispecific or multispecific antibody of the present disclosure. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, it is possible to produce E. coli, which has high industrial value due to low production cost, as a host cell.

The vector of the present disclosure may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer and may be variously produced according to the purpose. The promoter of the vector may be constitutive or inductive. The signal sequence may use a PhoA signal sequence, an OmpA signal sequence, etc. in the case of Escherichia sp. as a host, an α-amylase signal sequence, a subtilisin signal sequence, etc. in the case of Bacillus sp. as a host, an MFα signal sequence, a SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selective marker for selecting a host cell including a vector and a replicable expression vector includes a replication origin.

In the present disclosure, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (e.g., bacteriophages), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.).

In one embodiment, when constructing the vector, expression regulatory sequences such as a promoter, a terminator, and an enhancer, sequences for membrane targeting or secretion, etc. are appropriately selected according to a type of host cell to produce the antibody and may be variously combined depending on a purpose.

In the present disclosure, the term "expression vector" refers to a vector including a nucleic acid sequence encoding at least a portion of a gene product to be transcribed. In some cases, the RNA molecule is then translated into a protein, a polypeptide, or a peptide. The expression vector may include various regulatory sequences. In addition to regulatory sequences that regulate transcription and translation, vectors and expression vectors may also include nucleic acid sequences that provide other functions.

In the present disclosure, the term "host cell" includes eukaryotes and prokaryotes, and refers to any transformable organism capable of replicating the vector or expressing a gene encoded by the vector. The host cell may be transfected or transformed by the vector, which means a process in which an exogenous nucleic acid molecule is delivered or introduced into the host cell.

In one embodiment, the host cell may be bacteria or animal cells, the animal cell line may be a CHO cell, a HEK cell or a NSO cell, and the bacteria may be E. coli.

In one aspect, the present disclosure relates to a pharmaceutical composition for prevention or treatment of a disease or disorder selected from the group consisting of autoimmune disease, neurodegerative disease, Alzheimer's disease, metabolic disease, cardiovascular disease, atherosclerosis, organ transplant rejection, and diseases or symptoms caused by fungi, viruses, bacteria or parasites, including an antibody or an immunologically active fragment thereof, an antibody-drug conjugate, or a bispecific or multispecific antibody of the present disclosure as an active ingredient.

In one aspect, the present disclosure relates to a chimeric antigen receptor (CAR) including an antibody or an immunologically active fragment thereof of the present disclosure as an antigen binding domain.

In one aspect, the present disclosure relates to a recombinant vector including a gene encoding the chimeric antigen receptor.

In one aspect, the present disclosure relates to a chimeric antigen receptor expressing cell transformed with the recombinant vector.

In one embodiment, the chimeric antigen receptor expressing cell may be a chimeric antigen receptor expressing macrophage (CAR-macrophage), a chimeric antigen receptor expressing T (CAR-T) cell, a chimeric antigen receptor expressing-gamma-delta T (CAR-Gamma-delta T) cell, or a chimeric antigen receptor natural killer (CAR-NK) cell.

In the present disclosure, the term "chimeric antigen receptor (CAR)" refers to a non-naturally occurring receptor capable of imparting specificity for a specific antigen to an immune effector cell. Typically, the CAR refers to a receptor used to transplant the specificity of a monoclonal antibody into a T cell. The CAR usually consists of an ectodomain, a transmembrane domain, and an endodomain.

The ectodomain includes an antigen binding site, and the transmembrane domain of CAR is in a form linked to the ectodomain, and may be derived naturally or synthetically. When being derived naturally, the transmembrane domain may be derived from a membrane-binding or transmembrane protein, and may be a moiety derived from a transmembrane domain of various proteins, such as an alpha, beta or zeta chain of the T cell receptor, CD28, CD3 epsilon, CD45, CD4, CD5, CDS, CD9, CD16, CD22, CD33, CD37, CD64, CD80, CD86, CD134, CD137, CD 154 or CD8. The sequence of such a transmembrane domain may be obtained from literatures known in the art that disclose the transmembrane region of the transmembrane protein, but is not limited thereto.

In addition, when the transmembrane domain is synthesized, the transmembrane domain may mainly include hydrophobic amino acid residues such as leucine and valine, and examples thereof may be present in a transmembrane domain in which a triplet of phenylalanine, tryptophan, and valine is synthesized, but are not limited thereto. Sequence information for such a transmembrane domain may be obtained from literatures known in the art for the synthesized transmembrane domain, but is not limited thereto.

In the CAR of the present disclosure, the endodomain is a part of the domain of the CAR existing within a cell, and a form linked with the transmembrane domain. The endodomain of the present disclosure may include an intracellular signaling domain characterized by causing T cell activation, preferably T cell proliferation, when an antigen binds to an antigen binding site of the CAR. The intracellular signaling domain is not particularly limited in type as long as the intracellular signaling domain is a part that transmits a signal that may lead to T cell activation when an antibody binds to an antigen binding site existing outside the cell, and may be used with various types of intracellular signaling domains. For example, the intracellular signaling domain may be an immunoreceptor tyrosine-based activation motif or ITAM, and the ITAM may be derived from CD3 zeta (§), FcR gamma, FcR beta, CD3 gamma, CD3 delta, CD3 epsilon, CDS, CD22, CD79a, CD79b, CD66d or FcεRIγ, but is not limited thereto.

The CAR includes a single-chain fragment variable region (scFv) of an antibody specific to a tumor-associated antigen (TAA) that is coupled to the cytoplasmic domain of a T-cell signaling molecule via a hinge and a transmembrane region. Most conventional lymphocyte activating moieties include T-cell costimulatory domains (e.g., CD28, CD137, OX40, ICOS, and CD27) in a tandem with a T-cell triggering moiety (e.g., CD3ξ). CAR-mediated adoptive immunotherapy allows a CAR-grafted cell to directly recognize a TAA on a target tumor cell in a non-HLA-restricted manner.

In the present disclosure, the term "chimeric antigen receptor expressing T (CAR-T) cell" means a T cell expressing CAR. The CAR-T cell i) recognizes a cancer antigen in a manner independent of human leukocyte antigen (HLA) to have the advantage of being able to treat cancers that evade the action of anticancer drugs by reducing HLA expression on the cell surface, ii) is independent of HLA type to be used for treatment regardless of the patient's HLA type, and iii) has the advantage of being able to produce a large number of cancer-specific T cells in a short period of time to exhibit excellent anticancer effects.

The T cell includes CD4⁺ T cells (helper T cells, TH cells), CD8⁺ T cells (cytotoxic T cells, CTLs), memory T cells, regulatory T cells (Treg cells), natural killer T cells, etc., and the T cells into which the CAR is introduced in the present disclosure are preferably CD8⁺ T cells, but are not limited thereto.

In one embodiment, the present disclosure relates to a T-cell engager including an antibody or an immunologically active fragment thereof of the present disclosure.

In one embodiment, the T cell engager may be, for example, a bispecific T-cell engager (BiTE). The BiTE is a class of artificial bispecific monoclonal antibodies, which is a fusion protein consisting of amino acid sequences from four different genes or two single-chain variable region fragments (scFvs) of different antibodies on a single peptide chain of about 55 kilodaltons. One of the scFvs binds to a T cell via the CD3 receptor, and the other binds to a tumor cell via a tumor-specific molecule. Similarly to other bispecific antibodies, and unlike common monoclonal antibodies, the BiTE forms a linkage between a T cell and a tumor cell. The BiTE allows T cells to exhibit cytotoxic activity on tumor cells independently of the presence of MHC I or costimulatory molecules by producing proteins such as perforin and granzymes. These proteins are introduced into tumor cells and initiate apoptosis.

In one aspect, the present disclosure provides a pharmaceutical composition for prevention or treatment of cancer, including as an active ingredient an antibody or an immunologically active fragment thereof of the present disclosure, an antibody-drug conjugate, a bispecific or multispecific antibody, a chimeric antigen receptor, a chimeric antigen receptor expressing cell, or a T cell engager.

In one embodiment, the cancer may be any one selected from the group consisting of brain tumor, melanoma, myeloma, non-small cell lung cancer, oral cancer, liver cancer, gastric cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymphoma, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma, and may be cancer non-responsive to immune checkpoint inhibitors.

The pharmaceutical composition of the present disclosure may be used as a single therapy, but may also be used in combination with other conventional biological therapy, chemotherapy or radiation therapy, and may treat more effectively cancer in the case of such concurrent therapy. In the case of using the present disclosure for prevention or treatment of cancer, chemotherapeutic agents that may be used with the composition include cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, methotrexate, and the like. Radiation therapy that may be used together with the composition of the present disclosure includes X-ray irradiation and γ-ray irradiation.

In one embodiment, the composition of the present disclosure may further include an immunogenic apoptosis inducing agent. The immunogenic apoptosis inducing agent may be any one or more selected from the group consisting of anthracycline-based anticancer agents, taxane-based anticancer agents, anti-EGFR antibodies, BK channel agonists, Bortezomib, cardiac glycoside, cyclophosmide-based anticancer agents, GADD34/PP1 inhibitors, LV-tSMAC, Measles virus, bleomycin, mitoxantrone, or oxaliplatin. The anthracycline-based anticancer agent may be daunorubicin, doxorubicin, epirubicin, idarubicin, pixantrone, sabarubicin, or valrubicin, and the taxane-based anticancer agent may be paclitaxel or docetaxel.

The pharmaceutical composition for preventing or treating cancer of the present disclosure is administered together with a chemical anticancer drug (anticancer agent) and the like to increase a cancer treatment effect of conventional anticancer agents through the death effect of cancer cells. Combined administration may be performed simultaneously or sequentially with the anticancer agent. Examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics such as dactinomycin (actinomycin D), plicamycin, and mitomycin C; plant alkaloids such as vincristine, vinblastine, etoposide, teniposide, topotecan, and iridotecan; and the like, but are not limited thereto.

In the present disclosure, the term "prevention" means all actions that inhibit or delay the occurrence, spread, and recurrence of cancer by administration of the pharmaceutical composition according to the present disclosure.

In the present disclosure, the term "treatment" refers to all actions that improve or beneficially change the death of cancer cells or the symptoms of cancer by administration of the composition of the present disclosure. Those skilled in the art to which the present disclosure pertains will be able to determine the degree of improvement, enhancement and treatment by knowing the exact criteria of disease for which the composition of the present disclosure is effective by referring to data presented by the Korean Academy of Medical Sciences, etc.

In the present disclosure, the term "therapeutically effective amount" used in combination with the active ingredient means an amount of pharmaceutically acceptable salt of the composition effective for preventing or treating a target disease, and the therapeutically effective amount of the composition of the present disclosure may vary depending on many factors, such as a method of administration, a target site, the condition of a patient, and the like. Accordingly, when used in the human body, a dose should be determined as an appropriate amount in consideration of both safety and efficiency. It is also possible to estimate the amount to be used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective amount. In the present disclosure, the term "pharmaceutically effective amount" refers to an amount enough to treat the disease at a reasonable benefit/risk ratio applicable to medical treatment and enough not to cause side effects. The effective dose level may be determined according to factors including the health condition of a patient, the type and severity of cancer, the activity of a drug, the sensitivity to a drug, a method of administration, a time of administration, a route of administration, an excretion rate, duration of treatment, and drugs used in combination or simultaneously, and other factors well-known in the medical field. The composition of the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may further include pharmaceutically acceptable additives. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc, and the like. The pharmaceutically acceptable additive according to the present disclosure is preferably included in an amount of 0.1 parts by weight to 90 parts by weight based on the composition, but is not limited thereto.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present disclosure may be administered parenterally (e.g., applied with injectable formulations intravenously, subcutaneously, intraperitoneally or topically) or orally according to a desired method, and the range of the dose may vary depending on the body weight, age, sex, and health condition of a patient, a diet, an administration time, an administration method, an excretion rate, the severity of a disease, and the like. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present disclosure provides an anticancer adjuvant including as an active ingredient an antibody or an immunologically active fragment thereof of the present disclosure, an antibody-drug conjugate, a bispecific or multispecific antibody, a chimeric antigen receptor, a chimeric antigen receptor expressing cell, or a T cell engager.

In one embodiment, the anticancer adjuvant of the present disclosure may be administered concurrently, separately, or sequentially with a cancer immunotherapy.

In one embodiment, the anticancer adjuvant of the present disclosure may improve non-responsiveness to a cancer immunotherapy.

In one embodiment, the cancer immunotherapy may be immune checkpoint inhibitors, an immunosuppressant control drug, a cancer vaccine, an immunoadjuvant, an immune cell for cancer treatment, an immune cell activation cofactor, an antibody for cancer treatment, or cytokines required to maintain the activity of an immune cell for cancer treatment.

In one embodiment, the immunosuppressant control agent may be a drug that reduces the level of regulatory T cells (Treg).

In the present disclosure, the immune checkpoint inhibitor may be an inhibitor of CTLA-4, PD-1, PD-L1, PD-L2, LAG-3, BTLA, B7H3, B7H4, TIM3, KIR, TIGIT, CD47, VISTA or A2aR.

In one aspect, the present disclosure provides a composition for diagnosing cancer including as an active ingredient an antibody or an immunologically active fragment thereof of the present disclosure, an antibody-drug conjugate, a bispecific or multispecific antibody, a chimeric antigen receptor, a chimeric antigen receptor expressing cell, or a T cell engager.

In one embodiment, the marker may be a chromogenic enzyme, a radioactive isotope, a chromopore, a luminescent material, a fluorescent material, a probe or a tag, and the fluorescent material may be a cyanine (Cy)-based, Rhodamine-based, Alexa-based, BODIPY-based, or ROX-based fluorescent material, and may be Nile Red, 4,4-difluoro-4-bora-3a,4a-diaza-s-indacene (BODIPY), cyanine, fluorescein, rhodamine, coumarin or Alexa.

In one aspect, the present disclosure relates to a method for producing an antibody specific to EphA10 or an immunologically active fragment thereof, including a) culturing a host cell transformed with a vector including an isolated nucleic acid molecule encoding an antibody or an immunologically active fragment thereof of the present disclosure; and b) recovering a polypeptide expressed by the host cell.

In one aspect, the present disclosure relates to a method for producing a bispecific or multispecific antibody, including a) culturing a host cell transformed with a vector including an isolated nucleic acid molecule encoding a bispecific or multispecific antibody of the present disclosure; and b) recovering a polypeptide expressed by the host cell.

In one embodiment, the purification of the antibody may include filtration, HPLC, anion exchange or cation exchange, high performance liquid chromatography (HPLC), affinity chromatography, or a combination thereof, preferably affinity chromatography using Protein A.

In one aspect, the present disclosure relates to a use for the prevention or treatment of cancer of an antibody or an immunologically active fragment thereof of the present disclosure, an antibody-drug conjugate, a bispecific or multispecific antibody, a chimeric antigen receptor expressing cell, or a T cell engager.

In one aspect, the present disclosure relates to a method for treating cancer, including administering, to a subject suffering from cancer, an antibody or an immunologically active fragment thereof of the present disclosure, an antibody-drug conjugate, a bispecific or multispecific antibody, a chimeric antigen receptor expressing cell, or a T cell engager in a pharmaceutically effective amount.

In one aspect, the present disclosure relates to a use for the diagnosis of cancer of an antibody or an immunologically active fragment thereof of the present disclosure, an antibody-drug conjugate, a bispecific or multispecific antibody, a chimeric antigen receptor expressing cell, or a T cell engager.

### [Modes]

Hereinafter, the present disclosure will be described in more detail through the following Examples. However, the following Examples are only intended to embody the contents of the present disclosure, and the present disclosure is not limited thereto.

### Example 1. Expression and purification of EphA10 antigen protein

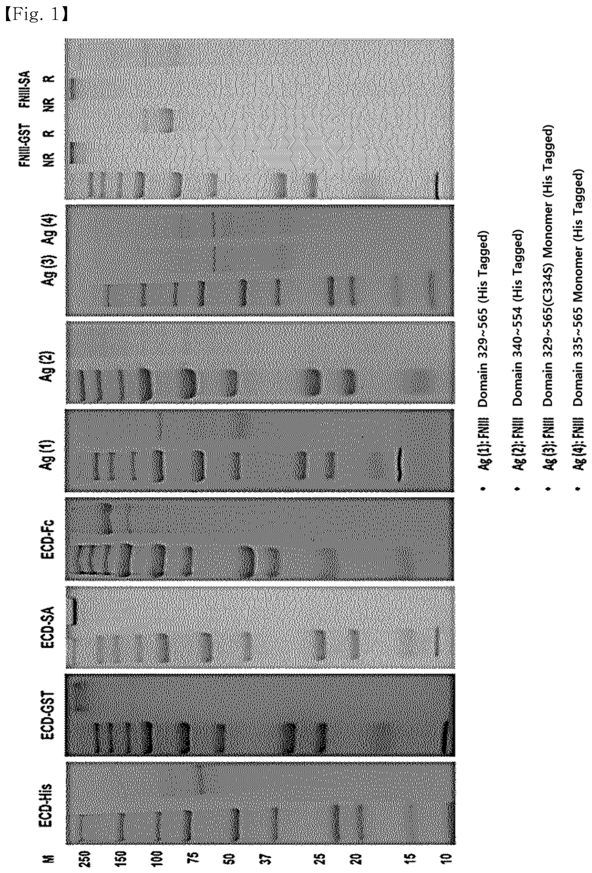

To produce an antigen for producing an antibody targeting Eph receptor A10 (EphA10), a cell membrane protein as a non-responsiveness inducing factor to a therapeutic agent targeting an immune checkpoint inhibitor, a vector for expressing an extra cellular domain (ECD) of EphA10 or a FNIII domain of the ECD was produced, and then expressed and purified in animal cells. To this end, a vector for animal cell expression of the ECD or FNIII domain of EphA10 was produced, and for more effective antibody screening by avidity, a vector for animal cell expression of a dimeric form of EphA10 protein fused with Glutathione-S-transferase (GST) or human Fc and a vector for animal cell expression of a tetrameric form of EphA10 protein fused with streptavidin was also produced. Specifically, Expi293F cells were subcultured at a density of 2 × 10⁶ cells/ml, and one day later, each vector produced above was transfected using PEI (Polyethylenimine, Polyscience, 23966). After shaking culture for 7 days in a CO₂ incubator at 37°C, 125 rpm, and 8% CO₂ conditions, the supernatant was collected by centrifugation, added with 25× PBS at 1/25 of the supernatant volume, and then filtered using a 0.2 µm bottle top filter (Merck Millipore). The filtered solution was added to 1 ml of GSH resin, Protein A resin, or Ni-NTA resin, stirred at 4°C for 16 hours, and then flowed through a column to recover each resin. The resin was washed with 5 ml of 1× PBS, and then eluted with 4 ml of 50 mM Tris-HCl (pH 8.0), 200 mM glycine (pH 2.7), or 250 mM imidazole containing 10 mM reduced glutathione, respectively. After replacing the buffer with 1× PBS using centrifugal filter units 3K (Merck Millipore), the sizes and purity of 10 purified antigen proteins were analyzed through SDS-PAGE (FIG. 1).

### Example 2. Acquisition and humanization of EphA10-binding mouse antibodies

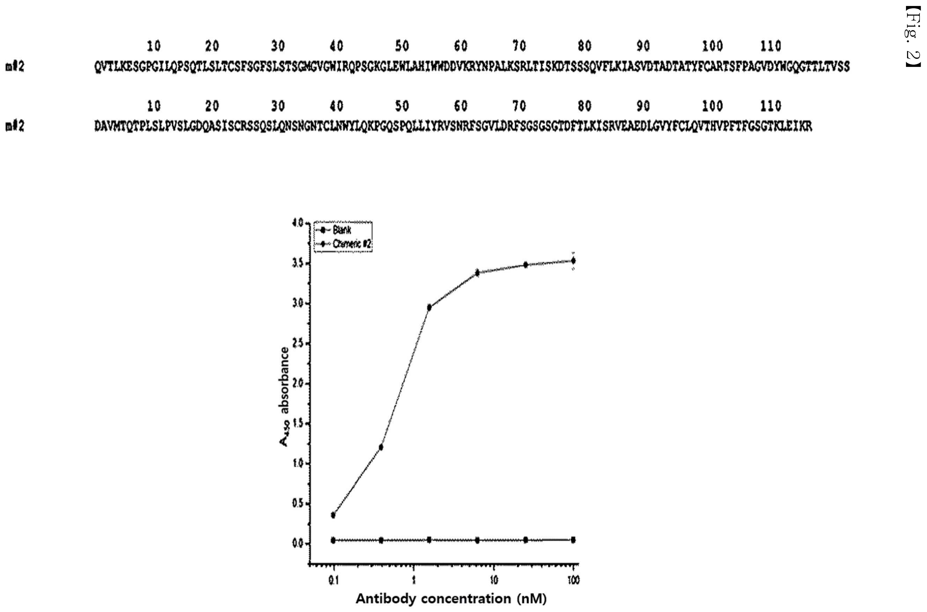

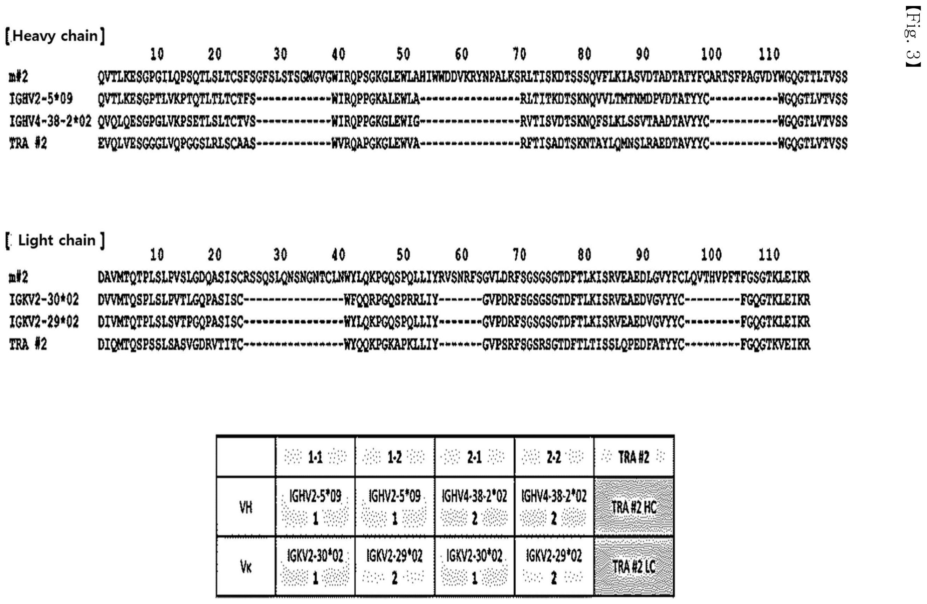

EphA10-specific mouse antibody m#2 was obtained by mouse immunization and hybridoma technology using the 10 antigens produced in Example 1 (Kohler G., Milstein C. Continuous cultures of fused cells secreting antibody of predefined specificity. Nature. 1975;256:495-497), and based on this, Chimeric #2 antibody introduced with a human constant region was obtained through animal cell expression and antibody purification (FIG. 2). To minimize the possibility of inducing immunogenicity by humanizing a sequence of a framework region of the produced EphA10-specific mouse antibody, germline sequencing of the EphA10-specific mouse antibody was performed. Through this, four types of EphA10-specific humanized antibodies 1-1, 1-2, 2-1, and 2-2 were obtained by introducing frameworks of two types of human heavy chains IGHV2-5*09 and IGHV4-38-2*02 and light chains IGKV2-30*02 and IGKV2-29*02 that had the highest similarity to the EphA10-specific mouse antibody, respectively. In addition, one humanized antibody TRA #2 was additionally produced by introducing a framework of trastuzumab, a humanized antibody with excellent physical properties, among therapeutic antibodies currently widely used in clinical practice (FIG. 3).

### Example 3. Expression and purification of EphA10-binding humanized antibodies

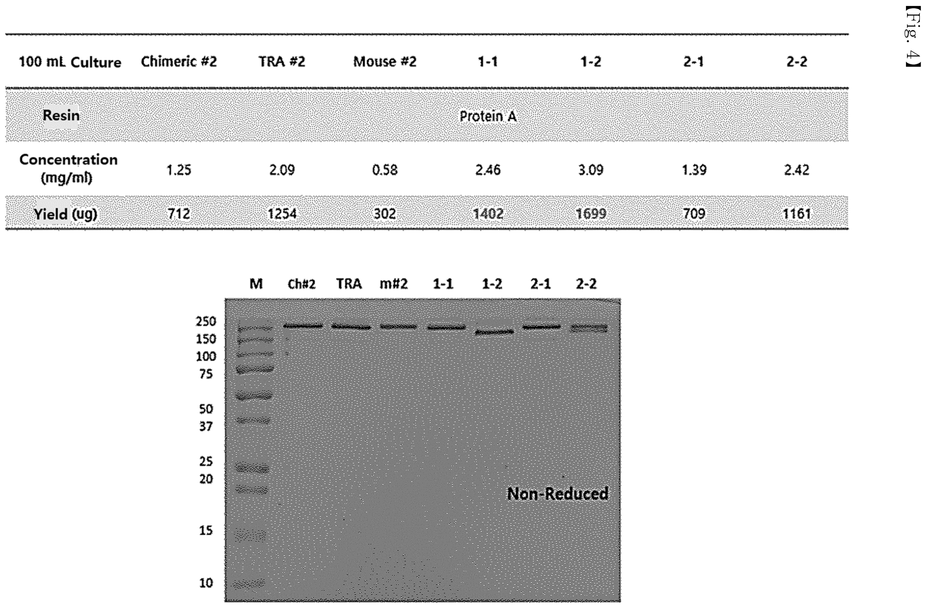

To express the five EphA10-specific humanized antibodies obtained in Example 2 above in IgG forms and analyze whether binding activity to EphA10 was maintained, three types of heavy chain and light chain expression vectors were produced. Expi293F cells were subcultured at a density of 2 × 10⁶ cells/ml, and one day later, transfected with the heavy chain expression vector and the light chain expression vector produced above into PEI (Polyethylenimine, Polyscience, 23966), and then shaking-cultured for 7 days in a CO₂ incubator at 37°C, 125 rpm, and 8% CO₂ conditions. After culturing, the supernatant was collected by centrifugation, added with 25× PBS at 1/25 of the supernatant volume, and then filtered using a 0.2 µm bottle top filter (Merck Millipore). The filtered solution was added with 500 µl of Protein A resin, stirred at 4°C for 16 hours, and then flowed through a column to recover a resin. The resin was washed with 5 ml of 1× PBS, eluted with 3 ml of 100 mM glycine (pH 2.7), and then neutralized using 1 M Tris-HCl (pH 8.0). After replacing the buffer with 1× PBS using centrifugal filter units 3K (Merck Millipore), the sizes and purity of the five purified antibodies were analyzed by SDS-PAGE (FIG. 4).

### Example 4. Selection of EphA10-specific humanized antibodies

### 4-1. Analysis of binding activity to EphA10

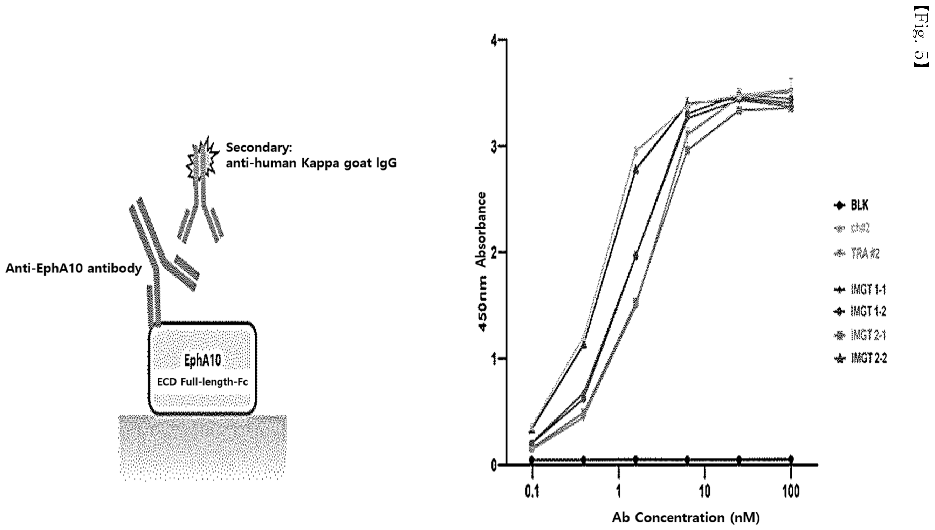

The binding activity to EphA10 of the five EphA10-binding humanized antibodies purified in Example 3 was analyzed by ELISA. Specifically, 4 µg/ml of EphA10 fused with human Fc was diluted with a 0.05 M Na₂CO₃ (pH 9.6) solution and immobilized in 50 µl of a Flat Bottom Polystyrene High Bind 96-well microplate (costar) at 4°C for 16 hours, and then blocked with 100 µl of 4% skim milk (GenomicBase) at room temperature for 2 hours. After washing four times with 180 µl of 1× PBS (PBST) containing 0.05% Tween 20, 50 µl of five antibodies IMGT 1-1, IMGT 1-2, IMGT 2-1, IMGT 2-2, and TRA #2 serially diluted in PBS containing 1% skim milk were dispensed into each well and reacted at room temperature for 1 hour. After washing, 50 µl of a goat anti-human kappa chain HRP conjugate (Millipore) was added, incubated at room temperature for 1 hour, and washed. 50 µl of a 1-Step Ultra TMB-ELISA substrate solution (Thermo Fisher Scientific) was added to develop color, and then added with 50 µl each of 2 M H₂SO₄ to terminate the reaction, and then analyzed using an Epoch microplate spectrophotometer (BioTek). As a result, it was confirmed that the humanized antibodies were successfully humanized without a significant decrease in binding activity to EphA10 compared to the mouse antibody ch#2 before the humanization process (FIG. 5).

### 4-2. Binding activity analysis and antibody selection for cell-expressing-EphA10

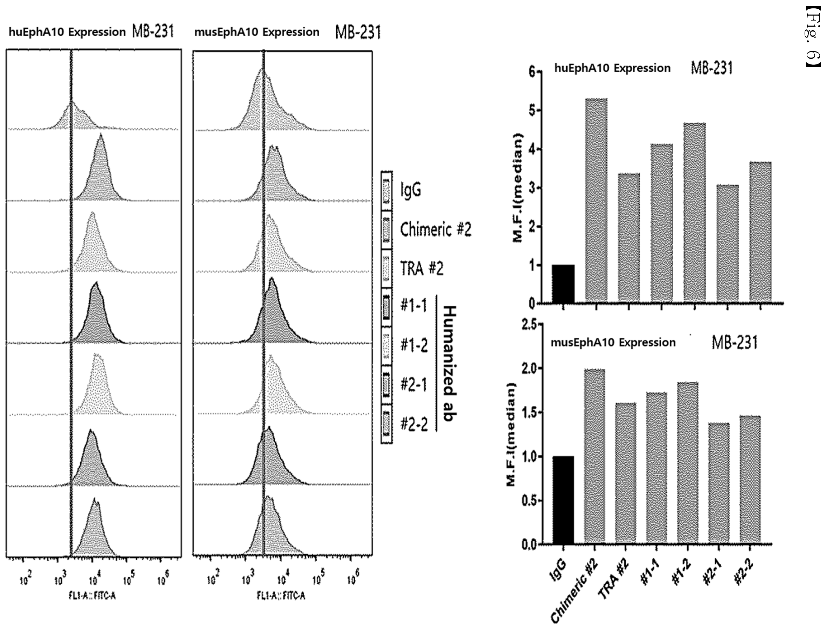

The binding activity of the five types of EphA10-binding humanized antibodies purified in Example 3 to cell-expressing-EphA10 was analyzed, and antibodies with strong binding activity were selected. Specifically, MDA-MB-231 human EphA10 overexpressing cell line and the mouse EphA10 overexpressing cell line were suspended with DPBS (Corning) and dispensed by 100 µl into each e-tube so that the number of cells was 9 × 10⁴. The cell lines were treated with 5 types of EphA10-binding humanized antibodies purified in Example 3 as a 1^{st} antibody and a mouse antibody Chimeric #2 and IgG before humanization as a control by each 500 ng and incubated at 4°C for 30 minutes, and treated with Alexa Fluor 488 anti-human IgG (Invitrogen) as a 2^{nd} antibody at a ratio of 1 : 100, and then incubated at 4°C for 30 minutes in a light-shielded manner. After washing twice with DPBS, the cell lines were analyzed using a CytoFLEX flow cytometer (Beckman Coulter). As a result, both the mouse antibody Chimeric #2 before humanization and the five humanized antibodies specifically bound to human and mouse EphA10, and all of the humanized antibodies were found to bind more strongly to human EphA10 than to mouse EphA10. In addition, among the five humanized antibodies, the humanized antibodies 1-1 and 1-2 showed the strongest binding activity to EphA10 and were selected as final candidates (FIG. 6).

### Example 5. Quantitative binding constant analysis of EphA10-specific humanized antibodies to EphA10

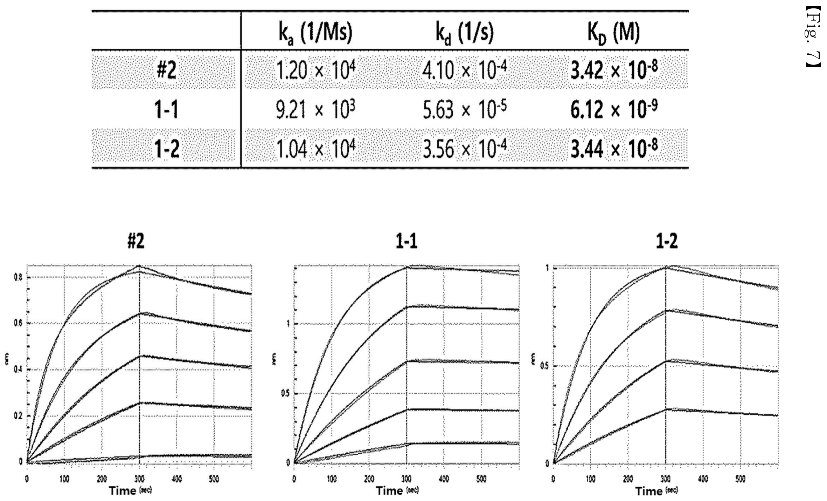

The quantitative binding constants of two selected EphA10-specific humanized antibodies 1-1 and 1-2 were analyzed using the Octet^{®} BLI system R8 (Satorius) as a BLI (Bio-Layer Interferometry) device. Specifically, for immobilization, 5 µg/ml of the two types of EphA10-specific humanized antibodies 1-1 and 1-2 diluted in 200 µl of 1× PBS were incubated on an Octet^{®} Protein A bio-sensor (Satorius) for 300 seconds. After baseline stabilization for 150 seconds using 1× PBS, EphA10-His monomeric antigen protein serially diluted in 1× PBS at a volume of 1,000 nM to 200 µl was associated with a biosensor coated with an EphA10-specific humanized antibody for 300 seconds. Thereafter, dissociation was performed by incubating 200 µl of 1× PBS for 300 seconds, and quantitative binding constants were analyzed using Octet BLI Analysis 12.2 software (Satorius) (FIG. 7).

### Example 6. Production of EphA10-targeting ADC antibodies

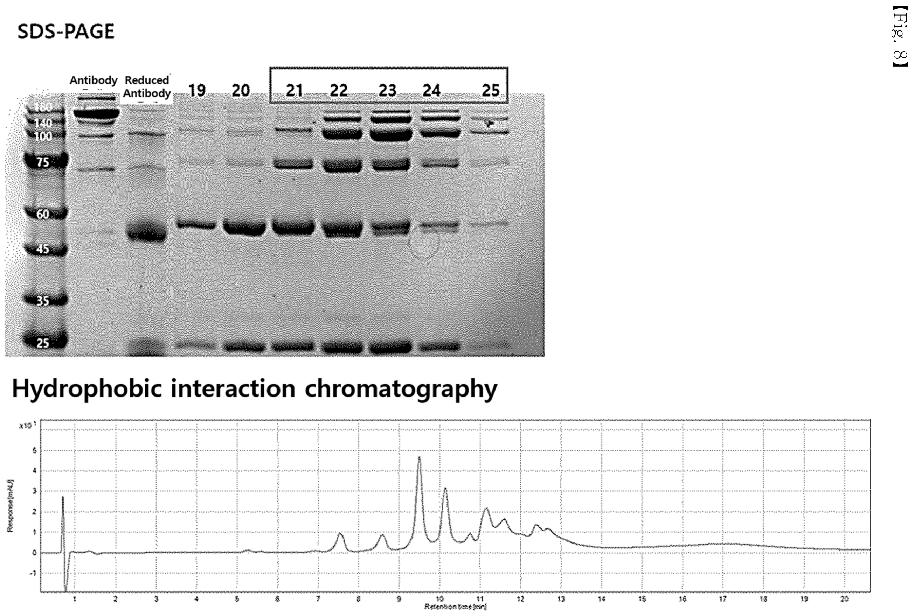

In order to produce an antibody drug conjugate (ADC) using the EphA10-specific humanized antibodies 1-1 and 1-2 selected in Example 4 above, the ADC was produced by a cysteine conjugation method followed by partial reduction, which has been generally used in producing the ADC. Specifically, the buffer of the EphA10-specific humanized antibodies 1-1 and 1-2, which were stored in 1× PBS, was exchanged with a reducing buffer (5 mM DTPA, pH 8.0 PBS) using PD MidiTrap G-25 (Cytiva, 28918008), and then enriched to 4 mg/ml using Centrifugal filter units 10K (Merck Millipore). 2 mg/ml of the enriched antibody and Tris(2-carboxyethyl)phosphine (TCEP, sigma) were mixed with the reducing buffer to be 68.7 µM, and the reduction reaction was performed at 37°C for 2 hours. Thereafter, TCEP was removed to prevent additional reduction, and for conjugation, exchange and enrichment were performed using PD MidiTrap G-25 column and Centrifugal filter units 10k in the same manner as the buffer exchange with 1× PBS buffer (pH 7.4). The reduced antibody at a concentration of 2 mg/ml was mixed with 63.2 µM MC-Val-Cit-PAB-MMAE (HY-15575, medchemexpress), a linker-drug reacting with cysteine, in 1× PBS and reacted at 4°C for 1 hour. To prevent additional reaction, cysteine was dissolved in 1× PBS and treated with 126.4 µM, which was twice the concentration of the treated linker-drug, and quenched at room temperature for 10 minutes. After size exclusion chromatography (SEC) purification was performed with the solution in which the reaction was completed, the reaction solution was injected into a Superdex 200 increase 10/300 (28990944, Cytiva) column in an FPLC (Biorad) system, and SEC chromatography was performed using 1× PBS at 0.4 ml/min, and then analyzed by SDS-PAGE (FIG. 8). The eluted ADC was enriched using Centrifugal filter units 10K and stored at -20°C until analysis and use, the purified ADC was analyzed by hydrophobic interaction chromatography, and the ADC peaks were separated by a difference in hydrophobicity that occurred according to DAR (FIG. 8).

### Example 7. Efficacy analysis and sequencing of EphA10-targeting ADC antibodies

### 7-1. Confirmation of cancer cell killing effect of ADC

To confirm the efficacy of ADC *in vitro,* a cancer cell killing effect was confirmed through cell survival analysis using ATP measurement. Specifically, MDA-MB-231 naive cells (control group), a mock cell line (negative control group), and a hEphA10 overexpressing cell line were dispensed at 2 × 10⁴ per well in a 96-well white plate (SPL), respectively, and the following day, the ADC produced in Example 6 was diluted in RPMI (Capricorn) for each concentration, treated by 100 µl per well, and then incubated at 37°C for 48 hours. The ADC-treated plate was incubated at room temperature outside the incubator for 5 minutes, 50 µl of a CellTiter-Glo solution was added to each well, and then the plate was shaken at 500 rpm for 2 minutes. After reacting for 10 minutes on a room temperature bench in a light-blocked state, ATP was measured using a Synergy HTX multimode reader (Biotek).

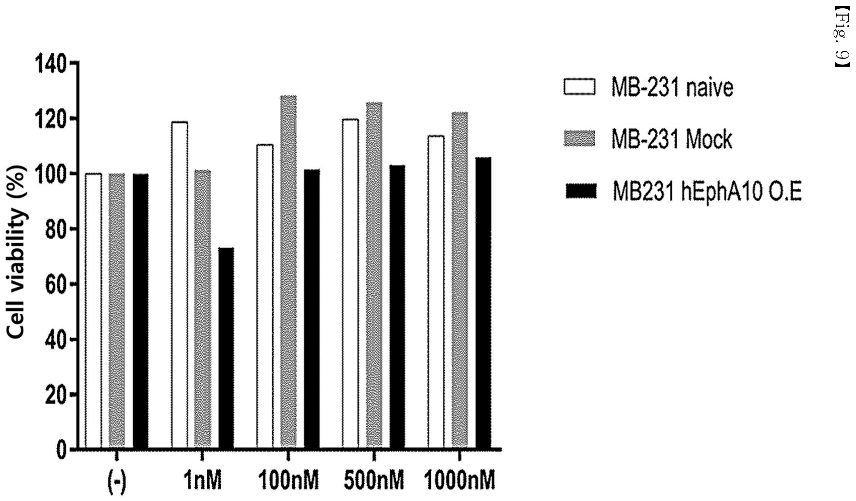

As a result, no clear cell killing effect was observed in the ADC (FIG. 9).

### 7-2. Sequencing

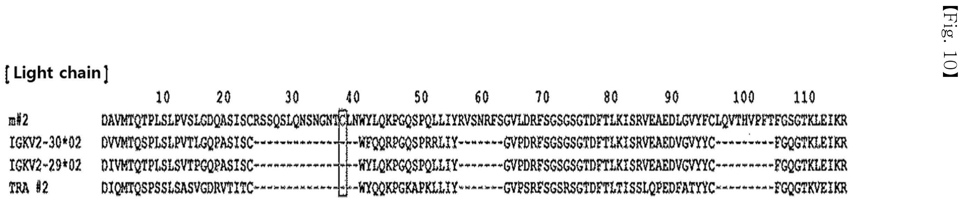

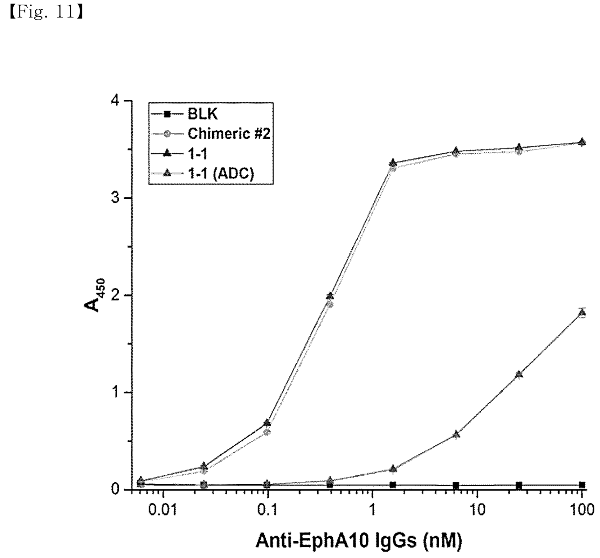

In order to analyze the reason why the ADC antibody using the EphA10-specific humanized antibody in Example 7-1 above did not show *in vitro* ADC efficacy, the sequence of the EphA10-specific humanized antibody was analyzed again. As the sequencing result, it was confirmed that a free cysteine amino acid that was not involved in disulfide bonds existed in the CDR region of the light chain of the EphA10-specific humanized antibody (FIG. 10). Since the EphA10-targeting ADC antibody was created by linking a low molecular material of payload through a linker, it was assumed that during this process, the payload was conjugated to the free cysteine in the CDR region to block a site binding to an antigen epitope of the EphA10-targeting humanized antibody, thereby losing antigen binding activity. To verify this, the binding activities of EphA10-specific humanized antibody 1-1 and EphA10-targeting ADC antibody 1-1 (ADC) after ADC production were analyzed and compared by ELISA. As expected, it was confirmed that the EphA10 binding activity of the ADC antibody was greatly reduced due to the free cysteine present in the CDR of the antibody light chain, and as a result, it was confirmed that the *in vitro* ADC efficacy was also greatly reduced (FIG. 11).

### Example 8. Production of EphA10-specific humanized antibodies with improved ADC efficacy

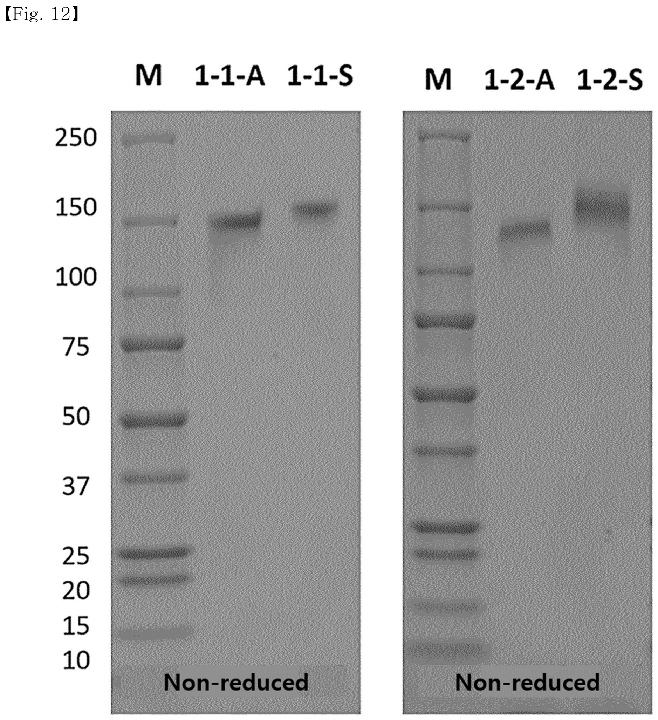

According to the results confirmed in Example 7 above, the free cysteines present in the light chains of the EphA10-specific humanized antibodies 1-1 and 1-2 selected in Example 4 were selected and replaced with serines 1-1-S and 1-2-S having similar properties to cysteine or alanines 1-1-A and 1-2-A to minimize the effect of the overall structural change of the antibody, respectively. Each humanized antibody was cloned into a vector for light chain animal cell expression, and each antibody was expressed and purified in animal cells as in Example 3 above, and the sizes and purity of the purified antibodies 1-1-A, 1-1-S, 1-2-A, and 1-2-S were analyzed through SDS-PAGE (FIG. 12).

### Example 9. Analysis of binding activity to EphA10 of EphA10-specific humanized antibodies with improved ADC efficacy

In Example 8 above, the binding activity to EphA10 of antibodies 1-1-A, 1-1-S, 1-2-A, and 1-2-S, in which the free cysteines present in the light chains of two types of EphA10-specific humanized antibodies 1-1 and 1-2 were substituted with serines and alanines, was analyzed by ELISA.

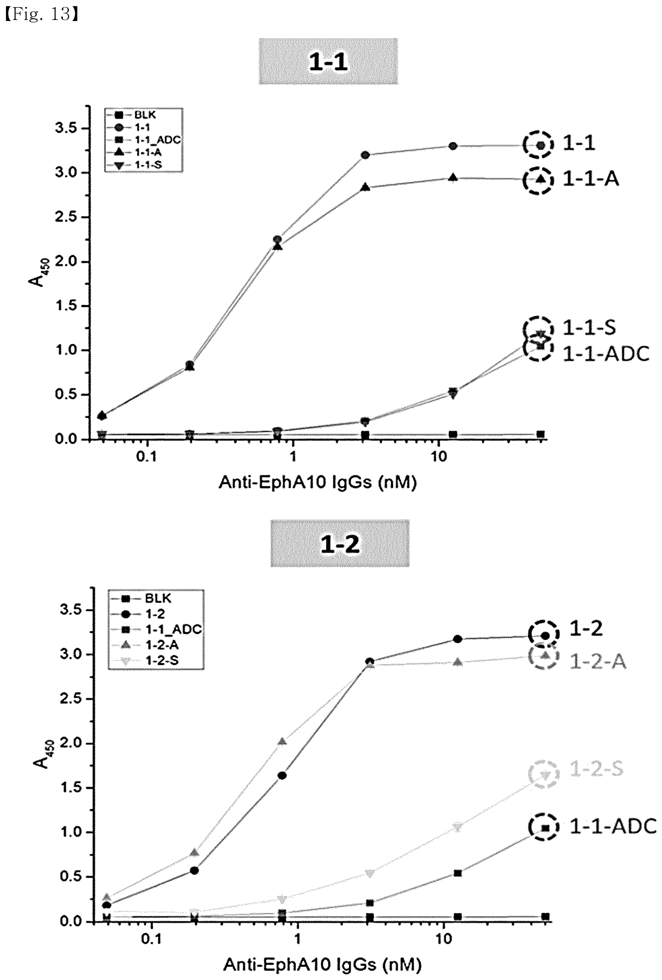

As a result, when the free cysteines of the EphA10-specific humanized antibodies 1-1 and 1-2 were replaced with serines, the binding activity to EphA10 was almost completely lost, whereas when the free cysteines were replaced with alanines, the binding activity to EphA10 was restored (FIG. 13).

### Example 10. Construction of yeast display library for improving EphA10 binding activity

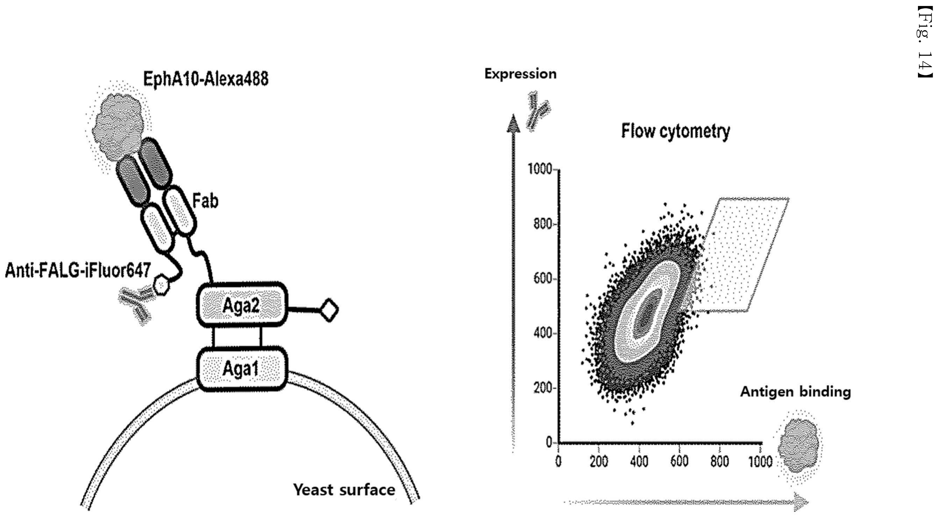

In order to discover antibodies with further improved binding activity to EphA10 from the EphA 10-specific humanized antibodies 1-1 and 1-2 discovered in Examples above, an antibody library was constructed by amplifying variable regions VH and VL of the antibodies 1-1 and 1-2 induced by introducing random mutations at 0.2% through error-prone PCR. The constructed DNA library was transformed into 400 µl of yeasts, Saccharomyces cerevisiae competent cells by electroporation using a MicroPulser Electroporator (Bio-Rad), and then cultured in 500 ml of a SD medium [Difco Yeast nitrogen base (BD Biosciences) 6.7 g/l, Bacto casamino acid (BD Biosciences) 5.0 g/l, Na₂HPO₄ (Junsei) 5.4 g/l, NaH₂PO₄.H₂O (Samchun) 8.56 g/l, Glucose (Duksan) 20 g/l]. Only the transformed yeast species were selectively survived, and a yeast display library with library sizes of 7.5 × 10⁷ and 1.0 × 10⁸ for 1-1 and 1-2 was produced, respectively (FIG. 14).

### Example 11. Screening of Humanized antibodies with improved binding activity to EphA10

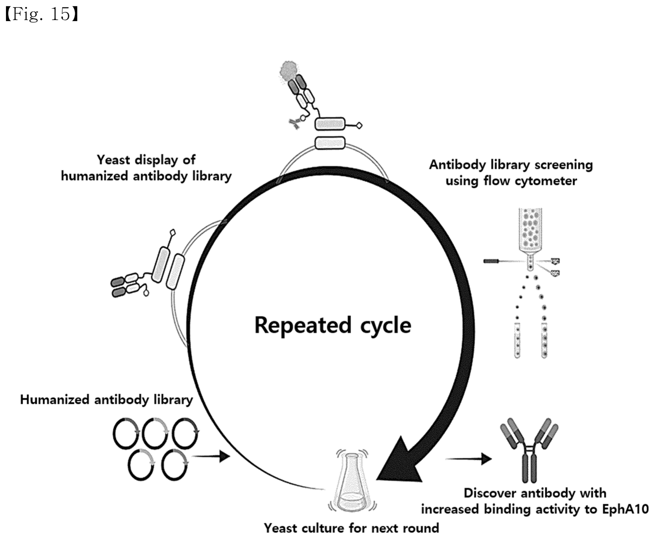

First, a human Fc-fused antigen protein EphA10-Fc was labeled with an Alexa488 fluorescent molecule for screening using a flow cytometer. In addition, 5.5 × 10⁸ cells of the yeast library produced in Example 10 were cultured in 100 ml of a SD medium lacking tryptophan at 30°C for 16 hours, and then 7 × 10⁸ cells were cultured in 100 ml of a SG medium [Difco Yeast nitrogen base (BD Biosciences) 6.7 g/l, Bacto casamino acid(BD) 5.0 g/l, Na₂HPO₄(Junsei) 5.4 g/l, NaH₂PO₄.H₂O (SAMCHUN) 8.56 g/l, Galactose (Sigma-Aldrich) 20 g/l] at 20°C for 48 hours. Among the cultured cells, 1 × 10⁸ cells were centrifuged at 14,000 × g for 30 seconds at 4°C to remove the supernatant, washed with 1 ml PBSB (1× PBS, 0.1% bovine serum albumin (Gibco) (pH 7.4), and then added with 200 nM Alexa488-conjugated EphA10-Fc and anti-FLAG-iFlour (GenScript) diluted in 1 ml of PBSB (pH 7.4) at a ratio of 1 : 1,000, and incubated for 1 hour. After incubation, the cells were washed with 1 ml PBSB (pH 7.4) and resuspended in 1 ml PBSB (pH 7.4) to induce binding of Alexa488-conjugated EphA10-Fc, and then cells showing high fluorescence signals were recovered through a flow cytometer (Bio-Rad S3 sorter, Bio-Rad). The recovered sub-library was subjected to a total of five rounds of selection by decreasing the concentration of Alexa488-conjugated EphA10-Fc in the same manner to enrich cells displaying Fab variants with high binding activity to EphA10 (FIG. 15).

### Example 12. Identification of genetic sequences of humanized antibodies with improved binding activity to EphA10

In Example 11 above, the gene sequences of Fab variant clones with excellent binding signals to EphA10 were confirmed by Sanger sequencing.

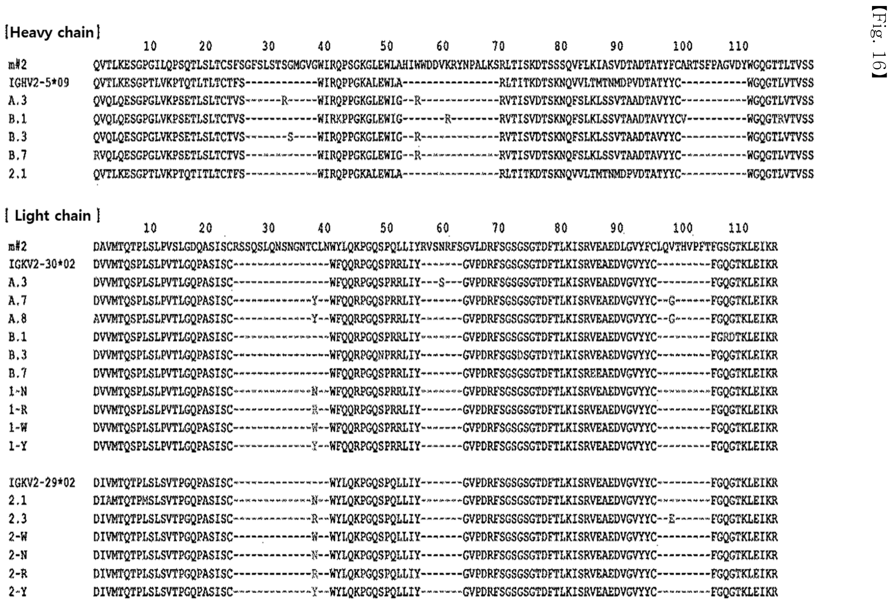

As a result of the analysis, it was confirmed that antibodies with specific antibody sequences were successfully enriched, and through screening for improving the EphA10 binding activity, 10 types of antibodies A.3, A.7, A.8, B.1, B.3, B.7, 2.1, 2.3, 1-N, and 2-W were obtained (FIG. 16). In addition, most clones of the discovered antibodies were identified to substitute a free cysteine present in the light chain CDR of the 1-1 and 1-2 antibodies with one of asparagine (N), arginine (R), tryptophan (W), or tyrosine (Y). Therefore, six variants 1-R, 1-W, 1-Y, 2-N, 2-R, and 2-Y were further produced by inducing single amino acid substitution mutations of the free cysteine present in the light chain CDR of the 1-1 and 1-2 antibodies to asparagine, arginine, tryptophan, or tyrosine, respectively (FIG. 16).

### Example 13. Expression and purification of humanized antibodies with improved binding activity to EphA10

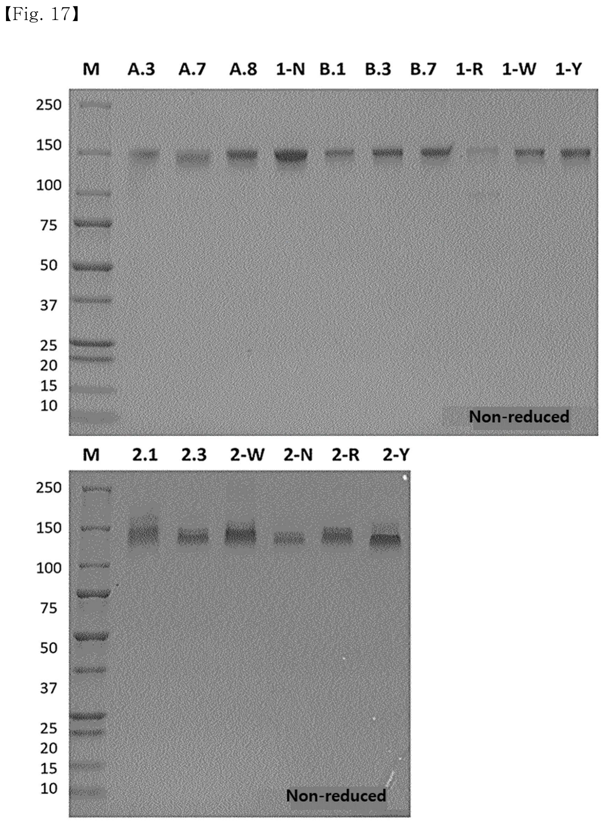

In order to confirm whether 16 Fab antibody variants selected in Example 12 above also had binding activity to EphA10 in the form of IgG, heavy chain and light chain expression vectors of the Fab antibody variants were produced, respectively, and each antibody was expressed and purified in animal cells as in Example 3, and then the size and purity of each of the 16 purified antibodies were analyzed through SDS-PAGE (FIG. 17).

### Example 14. Analysis of EphA10 binding activity of humanized antibodies with improved binding activity to EphA10

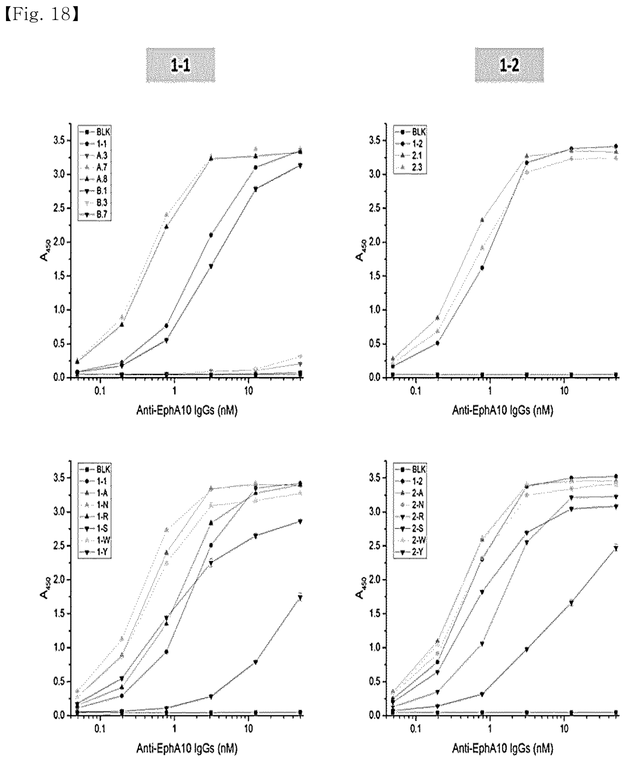

In order to confirm whether the binding activity to EphA10 of the 16 antibodies purified in Example 13 above increased, an ELISA analysis was performed, and as a result, it was confirmed that the binding activity to EphA10 of the humanized antibodies selected through additional engineering in Example 10 increased (FIG. 18).

### Example 15. Production of ADC of humanized antibodies with improved binding activity to EphA10

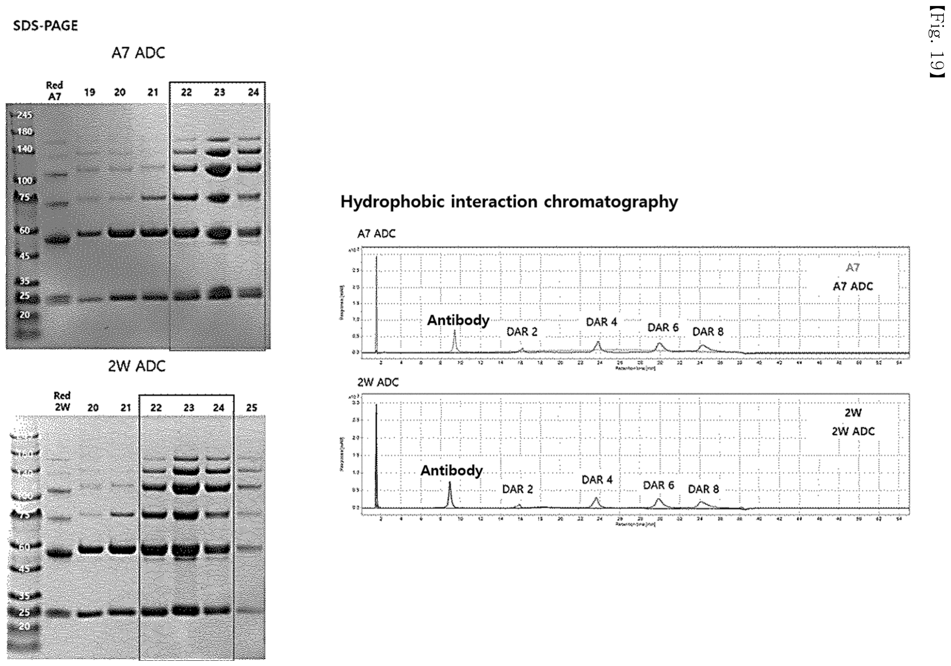

Among the EphA10-specific humanized antibodies selected after cysteine substitution in the CDR region and further engineering, Two types A.7 and 2-W were used to produce ADCs using the cysteine conjugation method of Example 6, and analyzed by SDS-PAGE (FIG. 19). Further, the purified ADCs were analyzed by hydrophobic interaction chromatography (FIG. 19). The produced ADC had peaks separated by a difference in hydrophobicity that occurred according to DAR, and the distribution and ratio of Drug to Antibody ratio (DAR) of the ADC were confirmed through HIC analysis.

### Example 16. Analysis of EphA10 antigen binding activity for humanized antibody-based ADC with improved binding activity to EphA10

It was analyzed by ELISA whether the EphA10 binding activity was maintained as before the ADC with respect of antibodies of EphA10-targeting ADC 1-1_ADC produced using the humanized antibody 1-1 selected in Example 4, the EphA10-targeting ADC 2W_ADC produced using an antibody 2-W in which the free cysteine present in the light chain CDR of the humanized antibody 1-2 selected in Example 4 was substituted with tryptophan, and the EphA10-targeting ADC A7_ADC produced using the antibody A7 A.7 selected through the yeast display library.

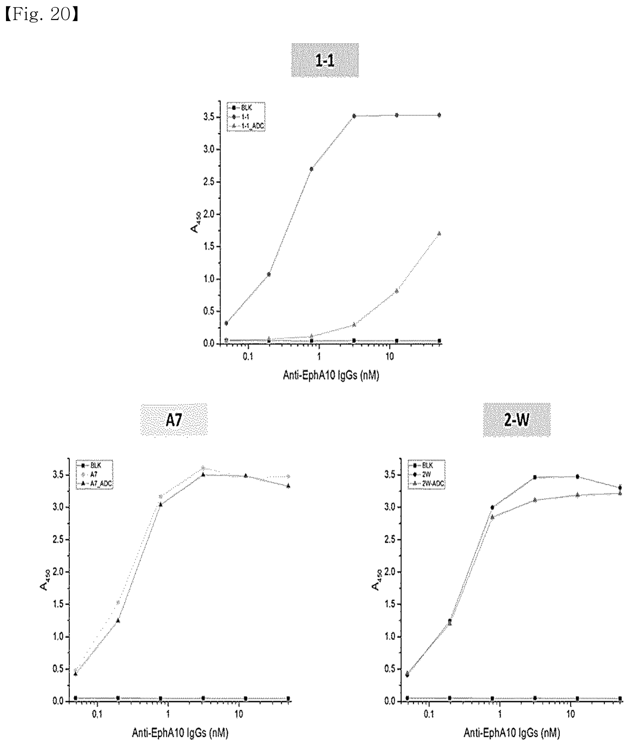

As a result, unlike a case where the binding activity to EphA10 was significantly reduced when the humanized antibodies 1-1 and 1-2 selected in Example 4 were produced as ADCs, it was confirmed that the binding activity to EphA10 was very well maintained when ADCs were produced from the selected antibodies by applying additional engineering (amino acid substitution, yeast display library) (FIG. 20).

### Example 17. Confirmation of cancer cell killing effect of humanized antibody-based ADCs with improved binding activity to EphA10

To verify the effect of two types of EphA10-targeting ADCs A7-MMAE and 2W_MMAE produced using A7 and 2W antibodies, cell viability analysis of cancer cells was performed by measuring ATP as in Example 7-1 above.

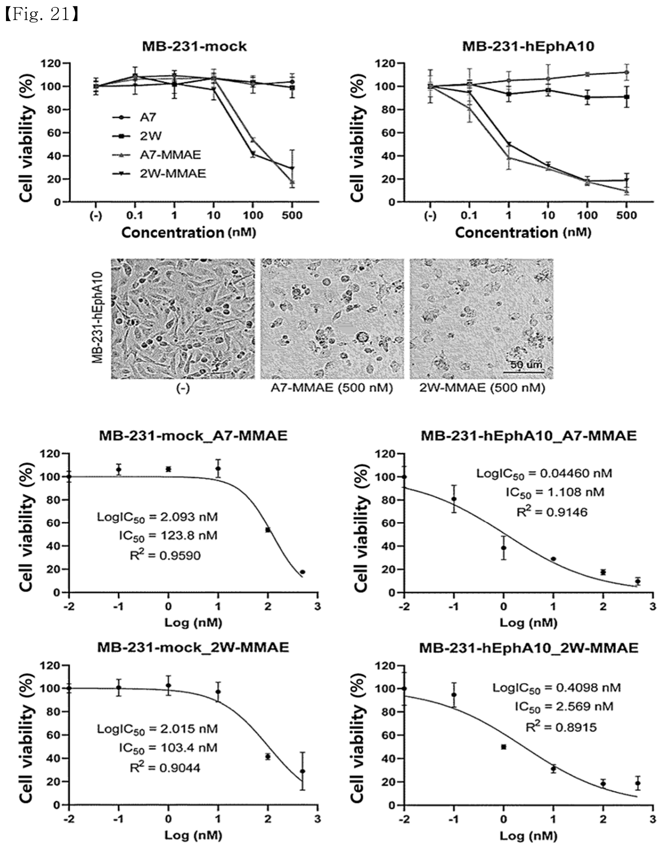

As a result, while the control antibodies A7 and W2 did not significantly affect cell survival, all of the EphA10-targeting ADCs using the antibodies clearly exhibited a cancer cell killing effect, and the cancer cell killing effect of the ADCs was found to increase in proportion to the expression of EphA10 (FIG. 21).

### Example 18. Quantitative binding constant analysis of humanized antibodies with improved binding activity to EphA10 to EphA10

To analyze the quantitative binding constants of two selected EphA10-specific humanized antibodies A.7 and 2-W to EphA10, experiments were performed using a Bio-Layer Interferometry (BLI) device, Octet^{®}BLI system R8 (Satorius). Specifically, for immobilization, 5 µg/ml of the two types of EphA10-specific humanized antibodies A.7 and 2-W diluted in 200 µl of 1× PBS were incubated on an Octet^{®} Protein A bio-sensor (Satorius) for 300 seconds. After baseline stabilization for 150 seconds using 1× PBS, a EphA10-His monomer antigen protein serially diluted in 1× PBS in a volume of 1,000 nM to 200 µl was bound to a biosensor coated with the EphA10-specific humanized antibodies A.7 and 2-W for 300 seconds. Thereafter, dissociation was performed by incubating 200 µl of 1× PBS for 300 seconds, and quantitative binding constants were analyzed using Octet BLI Analysis 12.2 software (Satorius).

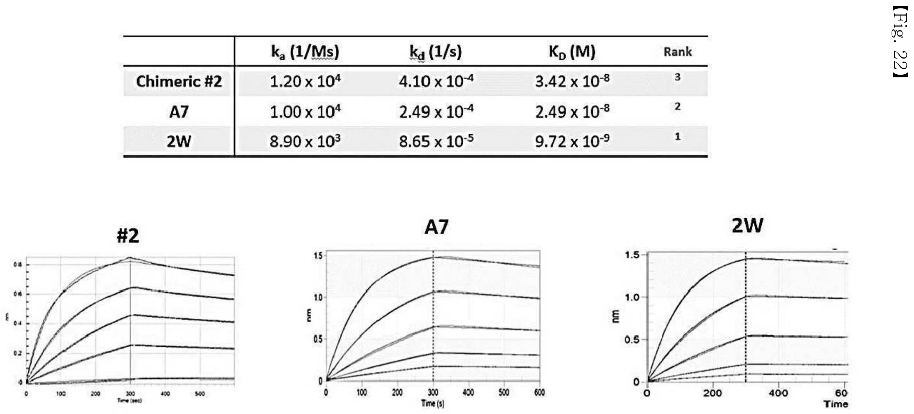

As a result, it was confirmed that both antibodies had higher binding constants to human EphA10 than antibody #2 (FIG. 22).

### Example 19. Analysis of mouse EphA10 binding activity of humanized antibodies with improved binding activity to EphA10

### 19-1. Expression and purification of mouse EphA10

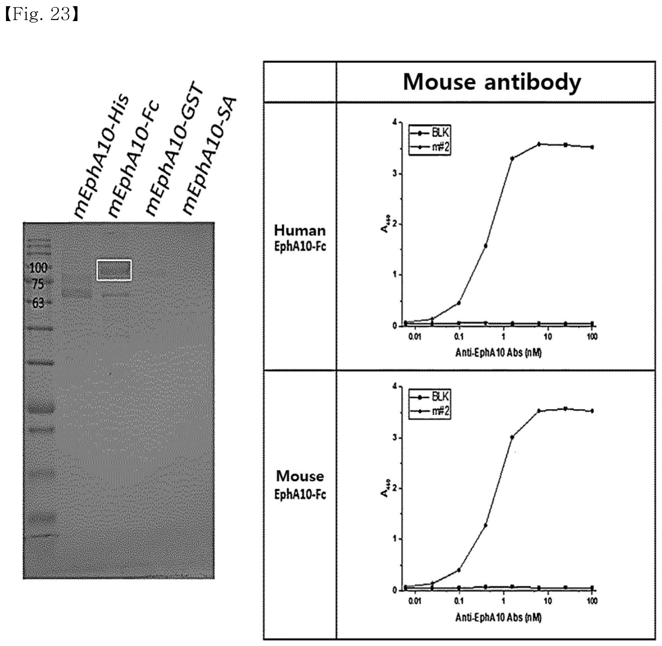

Since human EphA10 and mouse EphA10 had 95% similarity, an antibody binding to mouse EphA10 may be used as a good indicator for selecting a mouse model before *in vivo* experiments of EphA10-targeting antibodies. Accordingly, an animal cell expressing vector capable of expressing an extracellular domain (ECD) of mouse EphA10 together with various fusion proteins was prepared and transfected into Expi293F cells, and the cells were cultured for 7 days, and then centrifuged to collect the supernatant. The supernatant was added with 25× PBS at a 1/25 volume of the supernatant, and then filtered using a 0.2 µm bottle top filter (Merck Millipore), and the filtered supernatant was added with 1 ml of GSH resin, Protein A resin, or Ni-NTA resin, and then stirred at 4°C for 16 hours. Thereafter, the supernatant flowed through the column to recover each resin. The resin was washed with 5 ml of 1× PBS, and then eluted with 4 ml of 50 mM Tris-HCl (pH 8.0), 200 mM glycine (pH 2.7), or 250 mM imidazole containing 10 mM reduced glutathione, respectively. After replacing the buffer with 1× PBS using centrifugal filter units 3K (Merck Millipore), the sizes and purity of the four types of antigen proteins purified through SDS-PAGE were analyzed. As a result, among the four antigen proteins, only mEphA10-Fc (mouse EphA10 fused with human Fc) was purified, and the activity as an antigen thereto was confirmed by ELISA analysis using an EphA10-specific mouse antibody m#2 before the humanization process (FIG. 23).

### 19-2. Analysis of binding activity to mouse EphA10 of humanized antibodies with improved binding activity to EphA10

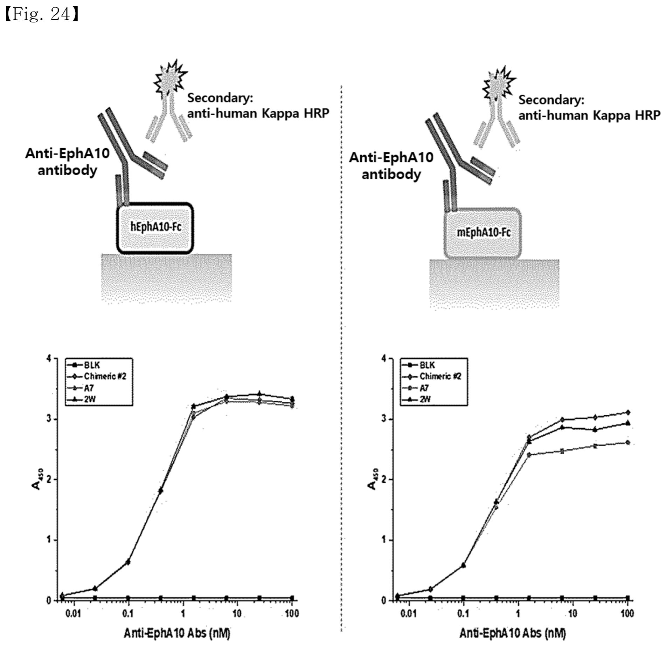

ELISA was performed to confirm whether the two selected EphA10-specific humanized antibodies A.7 and 2-W also bound to the mouse EphA10 expressed and purified in Example 19-1 above. Specifically, 4 µg/ml of mEphA10-Fc was diluted with a 0.05 M Na₂CO₃ (pH 9.6) solution and immobilized by 50 µl in a Flat Bottom Polystyrene High Bind 96-well microplate (costar) at 4°C for 16 hours, and then blocked with PBS containing 100 µl of 4% skim milk (GenomicBase) at room temperature for 2 hours. After washing four times with 180 µl of PBST, 50 µl each of EPHA10-binding antibodies A.7 and 2-W serially diluted in 1% skim milk was dispensed into each well and reacted at room temperature for 1 hour. After washing, 50 µl of a goat anti-human kappa chain HRP conjugate (Millipore) was added, incubated at room temperature for 1 hour, and washed. 50 µl each of a 1-Step Ultra TMB-ELISA Substrate Solution (Thermo Fisher Scientific) was added to develop color, and then added with 50 µl each of 2 M H₂SO₄ to terminate the reaction, and then analyzed using an Epoch microplate spectrophotometer (BioTek). As a result, it was confirmed that both the A7 antibody A.7 and the 2W antibody 2-W also bound to mouse EphA10 (FIG. 24).

## Claims

1. An antibody specific to Eph receptor A10 (EphA10) or an immunologically active fragment thereof.

2. The antibody specific to EphA10 or the immunologically active fragment thereof of claim 1, wherein the antibody specific to EphA10 or the immunologically active fragment thereof comprises a VH domain comprising a complementarity determining regions heavy chain (CDRH)1 including any one selected from the group consisting of amino acid sequences represented by SEQ ID NOS: 1 to 3, a CDRH2 including any one selected from the group consisting of amino acid sequences represented by SEQ ID NOS: 4 to 6, and a CDRH3 including an amino acid sequence represented by SEQ ID NO: 7 or 8.

3. The antibody specific to EphA10 or the immunologically active fragment thereof of claim 1, wherein the antibody specific to EphA10 or the immunologically active fragment thereof comprises a VL domain comprising a CDRL1 including any one selected from the group consisting of amino acid sequences represented by SEQ ID NOS: 9 to 14, a CDRL2 including an amino acid sequence represented by SEQ ID NO: 15 or 16, and a CDRL3 including any one selected from the group consisting of amino acid sequences represented by SEQ ID NOS: 17 to 19.

4. The antibody specific to EphA10 or the immunologically active fragment thereof of claim 1, wherein the antibody specific to EphA10 or the immunologically active fragment thereof comprises a VH domain including any one selected from the group consisting of amino acid sequences represented by SEQ ID NOS: 20 to 25.

5. The antibody specific to EphA10 or the immunologically active fragment thereof of claim 1, wherein the antibody specific to EphA10 or the immunologically active fragment thereof comprises a VL domain including any one selected from the group consisting of amino acid sequences represented by SEQ ID NOS: 26 to 45.

6. The antibody specific to EphA10 or the immunologically active fragment thereof of claim 1, wherein the immunologically active fragment is any one selected from the group consisting of Fab, Fd, Fab', dAb, F(ab'), F(ab')₂, single chain fragment variable (scFv), Fv, a single chain antibody, a Fv dimmer, a complementarity determining region fragment, a humanized antibody, a chimeric antibody, and a diabody.

7. The antibody specific to EphA10 or the immunologically active fragment thereof of claim 1, wherein the antibody is a humanized antibody.

8. An antibody-drug conjugate (ADC) comprising the antibody or the immunologically active fragment thereof of claim 1, and a drug.

9. The antibody-drug conjugate of claim 8, wherein the drug is immunogenic apoptosis-inducing agents, pro-apoptotic peptides, microtubulin structure formation inhibitors, mitosis inhibitors, topoisomerase inhibitors, DNA intercalators, toxins, radionuclides or anticancer agents.

10. The antibody-drug conjugate of claim 9, wherein the drug is selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof.

11. The antibody-drug conjugate of claim 8, further comprising:
an ADC linker.

12. The antibody-drug conjugate of claim 11, wherein the ADC linker is 6-maleimidocaproyl (MC), maleimidopropanoyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl) cyclohexane-1 carboxylate (SMCC), valine-citrulline-p-aminobenzyloxycarbonyl (val-cit-PAB), or N-succinimidyl (4-iodo-acetyl) aminobenzoate (SIAB).

13. The antibody-drug conjugate of claim 11, wherein the antibody or the immunologically active fragment thereof forms a complex with the drug via the ADC linker.

14. A bispecific or multispecific antibody comprising the antibody or the immunologically active fragment thereof of claim 1, and a moiety binding to a target antigen other than EphA10.

15. The bispecific or multispecific antibody of claim 14, wherein the moiety binding to the target antigen comprises an antibody or an immunologically active fragment thereof.

16. The bispecific or multispecific antibody of claim 14, wherein the target antigen is at least one selected from the group consisting of 17-1A antigen, GD3 ganglioside R24, EGFRvIII, PSMA, PSCA, HLA-DR, EpCAM, MUC1 core protein, aberrant glycosylation MUC1, fibronectin heteroform containing ED-B domain, HER2/neu, carcinoembryonic antigen (CEA), gastrin-releasing peptide (GRP) receptor antigen, mucin antigen, epidermal growth factor receptor (EGF-R), HER3, HER4, MAGE antigen, SART antigen, MUC1 antigen, c-erb-2 antigen, TAG 72, carbonic anhydrase IX, alpha-fetoprotein, A3, an antigen specific to an A33 antibody, Ba 733, BrE3-antigen, CA125, CD1, CD1a, CD3, CD5, CDl5, CD16, CD19, CD20, CD21, CD22, CD23, CD25, CD30, CD33, CD38, CD40, CD45, CD52, CD74, CD79a, CD80, CD138, colon-specific antigen-p (CSAp), CSAp, EGP-1, EGP-2, Ep-CAM, FIt-1, Flt-3, folate receptor, HLA-DR, human chorionic gonadotropin (HCG) and its subunits, hypoxia inducible factor (HIF-I), la, IL-2, IL-6, IL-8, insulin growth factor-1 (IGF-1), KC4-antigen, KS-1-antigen, KSl-4, Le-Y, macrophage inhibitory factor (MIF), MAGE, MUCl, MUC2, MUC3, MUC4, NCA66, NCA95, NCA90, antigen specific to PAM-4 antibody, placental growth factor, p53, prostatic acid phosphatase, PSA, RS5, SlOO, TAC, tenascin, TRAIL receptors, Tn antigen, Thomson-Friedenreich antigens, tumor necrosis antigens, VEGF, ED-B fibronectin, angiogenesis marker, oncogene marker or oncogene product.

17. The bispecific or multispecific antibody of claim 14, wherein the target antigen is a cell surface antigen or an autoantigen.

18. An isolated nucleic acid molecule encoding the antibody or the immunologically active fragment thereof of claim 1 or the bispecific or multispecific antibody of claim 14.

19. A pharmaceutical composition for prevention or treatment of a disease or disorder selected from the group consisting of autoimmune disease, neurodegerative disease, Alzheimer's disease, metabolic disease, cardiovascular disease, atherosclerosis, organ transplant rejection, and diseases or symptoms caused by fungi, viruses, bacteria or parasites, comprising as an active ingredient the antibody or the immunologically active fragment thereof of claim 1, the antibody-drug conjugate of claim 8, or the bispecific or multispecific antibody of claim 14.

20. A chimeric antigen receptor (CAR) comprising the antibody or the immunologically active fragment thereof of claim 1 as an antigen binding domain.

21. A recombinant vector comprising a gene encoding the chimeric antigen receptor of claim 20.

22. A chimeric antigen receptor expressing cell transformed with the recombinant vector of claim 21.

23. The chimeric antigen receptor expressing cell of claim 22, wherein the chimeric antigen receptor expressing cell is a chimeric antigen receptor expressing T (CAR-T) cell or a chimeric antigen receptor natural killer (CAR-NK) cell.

24. A T-cell engager comprising the antibody or the immunologically active fragment thereof of claim 1.

25. A pharmaceutical composition for prevention or treatment of cancer, comprising as an active ingredient the antibody or the immunologically active fragment thereof of claim 1, the antibody-drug conjugate of claim 8, the bispecific or multispecific antibody of claim 14, the chimeric antigen receptor of claim 20, the chimeric antigen receptor expressing cell of claim 22 or the T cell engager of claim 24.

26. The pharmaceutical composition for prevention or treatment of cancer of claim 25, wherein the cancer is any one selected from the group consisting of brain tumor, melanoma, myeloma, non-small cell lung cancer, oral cancer, liver cancer, gastric cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymphoma, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

27. The pharmaceutical composition for prevention or treatment of cancer of claim 25, wherein the cancer is cancer non-responsive to immune checkpoint inhibitors.

28. An anticancer adjuvant, comprising as an active ingredient the antibody or the immunologically active fragment thereof of claim 1, the antibody-drug conjugate of claim 8, the bispecific or multispecific antibody of claim 14, the chimeric antigen receptor of claim 20, the chimeric antigen receptor expressing cell of claim 22 or the T cell engager of claim 24.

29. The anticancer adjuvant of claim 28, wherein the anticancer adjuvant is administered concurrently, separately, or sequentially with a cancer immunotherapy.

30. The anticancer adjuvant of claim 28, wherein the anticancer adjuvant improves non-responsiveness to a cancer immunotherapy.

31. The anticancer adjuvant of claim 28, wherein the cancer immunotherapy is immune checkpoint inhibitors, an immunosuppressant control drug, a cancer vaccine, an immunoadjuvant, an immune cell for cancer treatment, an immune cell activation cofactor, an antibody for cancer treatment, or cytokines required to maintain the activity of an immune cell for cancer treatment.

32. The anticancer adjuvant of claim 31, wherein the immune checkpoint inhibitor is an inhibitor of CTLA-4, PD-1, PD-L1, PD-L2, LAG-3, BTLA, B7H3, B7H4, TIM3, KIR, TIGIT, CD47, VISTA or A2aR.

33. A composition for diagnosing cancer, comprising as an active ingredient the antibody or the immunologically active fragment thereof of claim 1, the antibody-drug conjugate of claim 8, the bispecific or multispecific antibody of claim 14, the chimeric antigen receptor of claim 20, the chimeric antigen receptor expressing cell of claim 22 or the T cell engager of claim 24.

34. The composition for diagnosing cancer of claim 33, further comprising:
a marker.

35. The composition for diagnosing cancer of claim 34, wherein the marker is a chromogenic enzyme, a radioactive isotope, a chromopore, a luminescent material, a fluorescent material, a probe or a tag.

36. A use for the prevention or treatment of cancer of the antibody or the immunologically active fragment thereof of claim 1, the antibody-drug conjugate of claim 8, the bispecific or multispecific antibody of claim 14, the chimeric antigen receptor expressing cell of claim 22 or the T cell engager of claim 24.

37. A method for treating cancer comprising:
administering, to a subject suffering from cancer, the antibody or the immunologically active fragment thereof of claim 1, the antibody-drug conjugate of claim 8, the bispecific or multispecific antibody of claim 14, the chimeric antigen receptor expressing cell of claim 22 or the T cell engager of claim 24 in a pharmaceutically effective amount.

38. A use for diagnosing cancer of the antibody or the immunologically active fragment thereof of claim 1, the antibody-drug conjugate of claim 8, the bispecific or multispecific antibody of claim 14, the chimeric antigen receptor of claim 20, the chimeric antigen receptor expressing cell of claim 22 or the T cell engager of claim 24.
